(19) **Europäisches Patentamt**
European Patent Office
Office européen des brevets

(11) **EP 2 090 229 B1**

(12) **EUROPÄISCHE PATENTSCHRIFT**

(45) Veröffentlichungstag und Bekanntmachung des Hinweises auf die Patenterteilung:
**02.11.2011 Patentblatt 2011/44**

(51) Int Cl.:
*A61B 8/12* (2006.01)     *G01B 17/06* (2006.01)
*G01N 29/22* (2006.01)     *G01S 15/89* (2006.01)

(21) Anmeldenummer: **09001941.5**

(22) Anmeldetag: **12.02.2009**

(54) **Anordnung und Verfahren zur Bestimmung der Kombination von Krümmungsradien und Abständen an akustischen Grenzflächen in Messobjekten mittels Ultraschall**

Assembly and method for determining the combination of curvature radii and distances to acoustic boundary areas in test objects with ultrasound

Agencement et procédé de détermination de la combinaison de rayons d'inclinaison et d'intervalles sur des surfaces limites acoustiques dans des objets de mesure à l'aide d'ultrasons

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **16.02.2008 DE 102008010582**

(43) Veröffentlichungstag der Anmeldung:
**19.08.2009 Patentblatt 2009/34**

(73) Patentinhaber: **Technische Universität Dresden 01062 Dresden (DE)**

(72) Erfinder:
• **Kühnicke, Elfgard**
  **01239 Dresden (DE)**
• **Trier, Hans-Georg**
  **53125 Bonn (DE)**

(74) Vertreter: **Hempel, Hartmut**
  **Patentanwaltskanzlei Hempel**
  **Jagdweg 10**
  **D-01159 Dresden (DE)**

(56) Entgegenhaltungen:
**WO-A-01/51888     US-A- 5 345 939**

• **BARSHAN BILLUR ET AL: "Radius of curvature estimation and localization of targets using multiple sonar sensors" JOURNAL OF THE ACOUSTICAL SOCIETY OF AMERICA, AIP / ACOUSTICAL SOCIETY OF AMERICA, MELVILLE, NY, US, Bd. 105, Nr. 4, 1. April 1999 (1999-04-01), Seiten 2318-2331, XP012000907 ISSN: 0001-4966**
• **AKHNAK M ET AL: "Development of a segmented annular array transducer for acoustic imaging" NDT & E INTERNATIONAL, BUTTERWORTH-HEINEMANN, OXFORD, GB, Bd. 35, Nr. 7, 1. Januar 2002 (2002-01-01), Seiten 427-431, XP004378480 ISSN: 0963-8695**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**EP 2 090 229 B1**

**Beschreibung**

Die Erfindung betrifft eine Anordnung und ein Verfahren zur Bestimmung der Kombination von Krümmungsradien und Abständen an akustischen Grenzflächen in Messobjekten mittels Ultraschall, wobei die Anordnung aus einem Pulsgenerator, der n>2 (mit n ε IN) gegeneinander zeitverzögerte Anregungssignale erzeugt, einer Sende-Empfangs-Weiche, einem Ultraschallwandler mit n>2 (mit n ε IN) Elementen zum Senden und Empfangen von Schallwellen, der die Signale vom Pulsgenerator über die Sende-Empfangs-Weiche erhält und dessen Elemente zeitverzögert angeregt werden und der von jedem Element aus eine zeitverzögerte Schallwelle in das Messobjekt zur Grenzfläche sendet und nach Umschaltung auf Empfang durch Schalter in der Sende-Empfangs-Weiche die von der Grenzfläche reflektierten Schallwellen separat an jedem Element empfängt, wobei zumindest durch den Ultraschallwandler und durch die Einschallrichtung zum Messobjekt eine Messanordnung vorgegeben ist, n>2 Verstärkungseinheiten, die die empfangenen Signale vom Ultraschallwandler über die Sende-Empfangs-Weiche erhalten, einer Aufnahmeeinheit, die mindestens einen Analog-Digital-Wandler zur Analog-Digitat-Wandlung der von der Sende-Empfangs-Weiche über die Verstärkungseinheiten weitergeleiteten Signale erhält, einer der Aufnahmeeinheit nachgeordneten Auswerteeinheit und einer Ergebnis-Anzeigeeinheit, die der Auswerteeinheit nachgeordnet ist, besteht. Solche Anordnungen sind in der Druckschrift Volker Deutsch, Michael Platte, Manfred Vogt: Ultraschallprüfung, Springer Verlag Berlin Heidelberg New York 1997, S.28/29 zur Fokussierung, S.55 zum Pulsgenerator, sowie in den Internatadressen http://focus.ti.com/docs/solution/folders/print/346.html, http://www.maxim-ic.com/ solutions/ultrasound/index.mvp, sowie http://www.analog.com/en/app/0.3174. 1001 %255F1167,00.html beschrieben.

Weitere Vorrichtungen basierend auf Anordnungen von Phasensynchronen Wandler ("phased arrays") werden in US5345939 und in Akhnak M. et al. : "Development of a segmented annular array transducer for acoustic imaging", NDT&E International 35 (2002), Bd. 35, Nr. 7, Seiten 427-431, ISSN: 0963-8695 beschrieben.

Ein System mit einem Messkopf bestehend aus fünf Wandlern, zur Bestimmung der Kombination von Krümmungsradien und Abständen mittels "time of flight" (TOF) Ultraschalltechnik ist aus Barshan Billur et al. : "Radius of curvature estimation and localization of targets using multiple sonar sensors", Journal of the Acoustical Society of America (April 1999), Seiten 2318-2331 bekannt.

Die Erfindung kann in folgenden Anwendungsbereichen eingesetzt werden:

In der Human- und Tiermedizin allgemein, soweit gekrümmte Grenzflächen an Gelenkflächen, Knochen, Organen und Hohlorganen, Blutgefäßen, Zysten und Aussackungen, umschriebenen Raumforderungen, Tumoren, und Formänderungen bei Bioimplantaten, z.B. durch Verformung infolge Muskelanspannung und durch Langzeit-Beanspruchung, vorhanden sind.

In der Augenheilkunde, wobei Messungen am Auge, insbesondere an den Grenzflächen von Homhaut, Linse, Glaskörper und Augenwand und von Bioimplantaten im Auge, z.B. Kunstlinsen. Grenzflächen von Gas- und Ol-Injektion, sowie von Implantaten mit eindellender Wirkung auf die Augenwand erfolgen. Insbesondere kann die Erfindung für Messungen am Augenhintergrund /und an der Linsenrückfläche eingesetzt werden, was zur Klärung des Akkommodationsvorgangs und zur Weiterentwicklung starrer und akkommodationsfähiger Linsenimplantate in der Augenchirurgie beiträgt. Alle Anwendungen, In der Medizin können eine genauere Diagnose, Behandlung und Kontrolle von Erkrankungen und operativen Eingriffen ermöglichen.

Bei der zerstörungsfreien Material-Prüfung kann eine Verbesserung der Bestimmung der Fehlergröße durch Berücksichtung der Krümmung sowie eine Verbesserung der Auflösung an gekrümmten Grenzflächen erreicht werden.

In der Verfahrenstechnik kann die Erfindung zur Kontrolle von gekrümmten Grenzflächen in Werkstoffen, Behältern und Rohren und in der Produktion eingesetzt werden.

Messobjekte können aus geschichteten Medien mit unterschiedlichem Konfigurationszustand bestehen und können sowohl Werkstoffe als auch biologische Gewebe aller Art sein. Im Einzelnen kann es sich um geschichtete Festkörper, ohne oder mit - auch gasgefüllten - Einschlüssen und Hohlräumen, welche wiederum typisch angelegt oder auch Artefakte bzw. Fehlbildungen sind; oder um eine geschichtete Struktur aus fluiden und/oder festen Medien, oder um eine bewegte Flüssigkeit mit sich verändernden Blasen handeln. In diese Gruppen fallen auch tierische oder menschliche Organe, z.B. das Auge. Wenn Artefakt und umgebendes Material bzw. zwei aneinandergrenzende Schichten unterschiedliche akustische Impedanzen besitzen, wird an den Grenzflächen zwischen den Materialien die Ultraschallwelle reflektiert oder gestreut, auch als akustisch wirksame Grenzfläche bezeichnet. Je nach Art des Messobjektes werden unterschiedliche Messanordnungen verwendet, die durch unterschiedliche Schallwandler, die Einschallrichtung und die Länge einer eventuell eingesetzten Wasservorlaufstrecke gekennzeichnet sind.

In den Anwendungsbereichen der Erfindung ist insbesondere von wissenschaftlichem oder technischem Interesse, gleichzeitig und ohne Veränderung der Messanordnung eine Kombination von geometrischen Kenngrößen, nämlich die Krümmungsradien der Grenzflächen in den Messobjekten und der Abstand der Grenzflächen von einer Bezugsebene des Schallwandlers zu erhalten.

Die herkömmlichen Methoden der Krümmungsradiusbestimmung setzen im Falle der optischen Verfahren ausreichend lichtdurchlässige Medien und im Falle von mechanischen oder gemischten optisch-mechanischen Verfahren die direkte Zugänglichkeit der Grenzfläche voraus.

Bei herkömmlichen Ultraschallverfahren werden A-mode-Vefiahren und B-mode-Verfahren unterschieden:

Das A-mode-Verfahren ist ein Impuls-Echo-Verfahren mit einem Einzel-Element Schallwandler, bei dem die Auswertung entweder am Hochfrequenz (HF)-Signal hinsichtlich Laufzeit, Phasen und Amplituden-Information oder nach einer AM-Demodulation unter Verzicht auf die Phasen erfolgt.
Das Verfahren ist ein eindimensional ausgerichtetes Verfahren, dessen Sendeinformatlonen konstant sind.
Bei A-mode-Verfahren dient die herkömmliche Auswertung der Ultraschall-Laufzeit zwischen einem Signalgeber und einem Signalempfänger und einer im Übertragungsweg befindlichen Grenzfläche eines Objektes zur Bestimmung des Abstandes der Grenzfläche vom Schallwandler.

Die Auswertung der Amplitude und Phasen des reflektierten Ultraschall-Signafs im A-mode-Verfahren dient zur Beurteilung der Höhe der durch Impedanzunterächiede und/oder Materialfeinstruktur bedingten Rückstreuung und ermöglicht Aussagen über Ort und Art des Einschlusses/Fehlers, der Delamination, der Art des angrenzenden Materials/ Gewebes. Sie ist aber bei Unkenntnis gekrümmter Grenzflächen mit hohen Fehlern behaftet Allgemein gilt, dass über die Krümmung keine direkten Informationen gewonnen werden können. Die Krümmung kann nur aus der Amplitude durch sukzessive Ortung von diversen Richtungen abgeschätzt werden. Dabei ist nur eine grobe Schätzung möglich, so dass bei gekrümmten Grenzflächen in Messobjekten meistens Fehlinterpretationen auftreten können.

Z.B. wird in der Druckschrift: Volker Deutsch, Michael Platte, Manfred Vogt: Ultraschallprüfung, Springer Verlag Berlin Heidelberg New York 1997, S.133ff für Messungen in der ZfP geschrieben: "Die Form des Fehlerechos lässt weitere Rückschlüsse vor allem auf die Obertlächengestalt der aufgefundenen Inhomogenitäten zu." Er führt aus, dass sich mit Hilfe der Echodynamik bei einer Schallkopfbewegung Fehlerformen unterscheiden lassen. Es wird weiterhin auf fehlerhafte Messungen, falsche Abstandsbestimmung und Fehlinterpretationen mit der Aussage hingewiesen, dass... Fehler mit unterschiedlichen Tiefen oder zum Teil schräg reflektierenden Oberflächenanteilen auch ein entsprechend "zerrissenes", verbreitertes und in der Amplitude reduziertes Echo..." hervorrufen.

Das B-mode-Verfahren stellt grundsätzlich eine Aneinanderreihung von A-mode-Zeilen dar, typisch jedoch in Helligkeits- statt Amplitudenmodulation, und ist ein Abtastverfahren, das manuell oder automatisch mit einem Einzelelement-Schallwandler oder einem Multielement-Schallwandler durchgeführt wird. Die Sendeinbrmatlonen am Einzelelement-Schallwandier sind konstant, während bei einem Linien-Array eine variable elektronische Schwenkung und/ oder Fokussierung des Ultraschallbündels vorgesehen sein kann. Durch die Nutzung der topografischen Informationen der Z mode-Zeilen als Empfangsgrößen wird ein Teil der Oberflächenkontur sichtbar.

Die Kontur einer Grenzfläche lässt sich daher anhand ihrer Reflexion und Rückstreuung grob, jedoch nur in einer Schnittebene pro Abtastvorgang abschätzen. Um die Grenzfläche grob räumlich dreidimensional zu beurteilen, sind mit dem B-mode-Verfahren sukzessiv zahlreiche Schnittbilder in anderen Gradlagen - in Meridianen - nötig. Durch die jeweils erforderliche Neujustierung ist die Konsistenz solcher Daten aber beschränkt Es treten dabei Justierfehler auf, die auf das sukzessive Einstellen mehrerer Ebenen mit nicht koinzidenten Achsen zurückzuführen sind. Dieser Nachteil entfällt zwar bei Einsatz von Schallwandlern mit zweidimensionaler Matrix von Elementen, so genannten 3D-Schallköpfen.

Die Druckschrift Hertz, C.H.: Pulse-echo techniques, Quality of linear and sector scan images and the compound scan. Handbook of clinical ultrasound. Ed: de Vlieger et al., John Wiley & Sons, New York 1978, page 35-36 beschreibt die typischen Abbildungsfehler bei der B-mode Abtastung einer Kugel und eines Zylinders. Die groben Artefakte von Linear- und Sektorabtastung mit einem Einzel-Wandler verringem sich zwar durch eine Compound-Abtastung, d.h. wenn die Grenzfläche aus mehreren Richtungen und Winkeln geortet wird und die Ergebnisse überlagert werden, jedoch verbleiben Artefakte in Form von "Rosetten" anstatt der wahren kreisförmigen Querschnitte. Elektromechanische Schallköpfe sind meistens auf Sektorabtastung festgelegt und daher für die Abbildung konvexer Krümmungen ungeeignet wie in der Druckschrift Thijssen, J.M.; A new B-scan system for ophthalmic diagnosis. Ophthalmic Research 2, 285-294,1971 beschrieben ist

Ähnliche Beschränkungen gelten grundsätzlich auch für Muldelement-Wandier. Über die Bauform dieser Wandler, bei denen die Wandlerelemente auf ebenen, konvexen oder konkaven Trägern angeordnet sind, kann nur die Abbildung bestimmter passender Krümmungen optimiert werden Die Konturdarstellung wird durch Nebenkeulen und Speckle beeinträchtigt. Die für die örtliche Auflösung wichtige effektive Bündelbreite kann zwar in der Schnittebene variiert, d.h. auf eine untersuchte Ebene eingestellt werden, jedoch bei einzeiligen Ausführungen nicht in der azimutalen Richtung, sodass die abgebildete Kontur in jedem Meridian Mittelungsfehler aufweist, wie in der Druckschrift Klews, P.-M.: Physik und Technik der Ultraschalldiagnoseverfahren, Moderne Bildgebung, Ed. Ewen,K., Thieme, Stuttgart, New York 1998, page 197-221 beschrieben ist.

Probleme beider Verfahren bestehen darin, dass sie keine exakte Bestimmung des Krümmungsradius von Grenzflächen erlauben, da die Genauigkeit der Ortsdefinition durch mehrere Faktoren, u.a. Bündelbreite, laterale und azimutale Empfängerauflösung, Nebenkeulen und Artefakte, begrenzt wird.

Sowohl beim A- als auch beim B-mode-Verfahren wird, wenn die Grenzflächengröße in der Größenordnung der Bündelbreite ist, die Krümmung nicht sichtbar.

Beide Verfahren haben auch allgemein das Problem, dass krümmungsbedingte Signalformänderungen und Änderungen der Signalhöhe zu Fehlem bei der Bestimmung des Ortes und der Größe der Ortskrümmung der Grenzfläche und zu falschen Aussagen bei der Bewertung des Grenzübergangs führen können, z.B. können bei konkav gekrümmten Grenzflächen ähnliche Signalformen wie bei einer Delamination oder einem Hohlraum entstehen.

Es ist eine Einrichtung zur Bestimmung des Krümmungsradius und zur Lokalisierung von Objekten mit Hilfe mehrerer Ultraschallsensoren in der Druckschrift B. Barshan et al. Radius of curvature estimation and localization of targets using multiple sonar sensors, J. Acoust. Soc. Am., vol. 105, No. 4, April 1999, Seiten 2318 bis 2331 beschrieben, wobei von einem Pulsgenerator ausgehend ein nachgeordneter Sender Signale auf ein Messobjekt übermittelt dessen reflektiertes Signal von einem Empfänger des Ultraschallwandlers aufgenommen und einer computergestützten Auswerteeinheit zugef-uhrt wird.

Die Anordnung enthält fünf Ultraschallwandler, die in größeren Abständen zueinander und zum Messobjekt angeordnet sind. Die Ermittlung des Abstandes und des Krümmungsradius eines reflektierenden Objektes in einer Flüssigkeit oder in einem Gas wird mit Hilfe eines Triangulationsverfahrens aus den Laufzeiten der reflektierten Ultraschallsignale an den verschiedenen Wandlern bestimmt Die Wandleranordnung ist z.B. für den Einsatz als bewegtes Navigationssystem für einen auf dem Meeresgrund fahrenden Roboter entwickelt worden. Die Genauigkeit des Verfahrens hängt von den Laufzeitunterschieden der an den verschiedenen Wandlern ankommenden Welle und damit vom Abstand des reflektierenden Objektes, vom Abstand der Ultraschallwandler und von der Winkelorientierung des Objektes bezüglich der Wandleranordnung ab. Das Verfahren ist mit den gleichen Fehlern wie die A-Bild-Ortung behaftet. In der Einrichtung wird beschrieben, dass die Genauigkeit der Messungen durch eine Optimierung des Abstandes zwischen den Wandlern entsprechend dem vorliegenden Abstand des Objektes und durch eine zusätzliche Rotation der Ultraschallwandleranoninung erhöht wird.

In der genannten Einrichtung wird eine Untersuchung von mehrschichtigen Messobjekten bzw. eine Krümmungsbestimmung von verdeckten Strukturen nicht angestrebt. Das Verfahren ist zur Entfemungs- und Krümmungsbestimmung eines in einer Flüssigkeit liegenden Objektes anwendbar, aber nicht für geschichtete Festkörper und medizinische Gewebe, bestehend aus mehreren Gewebsarten, z.B. beim Auge, geeignet Für eine Anwendung in der Verfahrenstechnik bei fluiden Medien ist die Wandleranordnung auch nur bedingt geeignet, da sie in der Flüssigkeit bewegt wird, von verschiedenen Positionen Messdaten sammelt und damit ein Eingriff in den Prozess erfolgt. Außerdem stellt der notwendige große Abstand zwischen den Wandlern bei kleinen Distanzen ein weiteres Problem dar, da durch die große Raumforderung eine Ankopplung an kleine Objekte nicht möglich ist.

Der Erfindung liegt deshalb die Aufgabe zugrunde, eine Anordnung und ein Verfahren zur Bestimmung der Kombination von Krümmungsradien und Abständen an akustischen Grenzflächen in Messobjekten mittels Ultraschall anzugeben, die derart geeignet ausgebildet sind, dass der Krümmungsradius mit hoher Genauigkeit besijmmt und damit nachfolgend als Basis-Parameter für die angegebenen Anwendungsgebiete eingesetzt werden kann.

Die Aufgabe der Erfindung wird durch die Merkmale der Patentansprüche 1 und 13 gelöst.

Weitenbildungen und spezielle Ausgestaltungen den Erfindung sind in weiteren Abhängigen Ansprüchen beschrieben.

Die Vorauswahl eines Kreisring-Ultraschallwandlers erfolgt entsprechend folgenden Gesichtspunkten:

A Mit ringförmigen Elementen, Ek lässt sich ein Punktfokus erzielen, wohingegen linienförmig Elemente einen Linienfokus, der in einer Richtung nicht fokussierbar ist, erzeugen. Um einen Punktfokus zu erzeugen, und aus Symmetriegründen beim Vorliegen von sphärischen oder torischen Grenzflächen, werden ringförmige Elemente Ek eingesetzt Die ringförmigen Elemente Ek erweisen sich auch beim Empfang als vorteilhaft, da bei den reflektierten Schallwellen die registrierten Schalldruckunterschiede auf den verschiedenen Elementen Ek, wenn sie als Ringe ausgelegt sind, besonders groß sind. Das verbessert die Genauigkeit der Krümmungsradiusbestimmung.

B. Zur Bestimmung der räumlichen Lage der Grenzfläche, zur Justierung und zur Erfassung einer torischen Krümmung können einzelne Elemente des Ultraschallwandlers in radialer Richtung zusätzlich segmentartig unterteilt sein.

C. Für die Wahl der Elementzahl und der Elementgröße ergibt sich Folgendes:

Der Fokussierungsbereich eines Ultraschallwandlers hängt von seiner Gesamtgröße, seiner Mittenfrequenz und der Breite der Einzelelemente Ek ab. Damit die notwendige Elementeanzahl nicht zu groß wird und die Fokussierung trotzdem in der Umgebung der Grenzfläche erfolgen kann, werden deshalb für verschiedene Anwendungen in Abhängigkeit des Abstandes $a_1$ der Grenzfläche vom Ultraschallwandler unterschiedliche Ultraschallwandler eingesetzt. Die Verwendung von Elementen Ek gleicher Fläche erweist sich als zweckmäßig für eine bessere Fokussierung. Außerdem lässt sich durch den Einsatz eines gekrümmten Sender-/Empfängerschallwandlers oder durch Anpassung der Ringabstände im Schallwandlerbereich die notwendige Anzahl der Ringelemente reduzieren. Im Empfangsbereich sind meistens weniger Elemente als im Sendebereich zur Krümmungsradiusbestimmung erforderlich. Durch die zusätzlich vorhandenen Elemente Ek lässt sich durch eine Mittelung aber die Genauigkeit der Krümmungsradienbestimmung erhöhen.

Bei konstant bleibender Krümmung lassen sich die-Empfangselemente nacheinander bei Wiederholung des Sendesignals auswerten und somit ist nur ein Analog-Digital-Wandler erforderlich. Für schnellere Auswertungen und bei dynamischen Vorgänger sind alle Elemente Ek einzeln auszuwerten, wobei pro Element Ek ein Analog-Digital-Wandler erforderlich ist.

Bei dynamischen Vorgängen, bei denen Bewegung und/oder Formveränderung vorlieben, wie z.B. Krümmungsmessung und Abstandsmessung an sich bewegenden, wachsenden und sich in der Form verändernden Blasen oder der Formänderung von biologischen Organen, etwa durch kreislaufbedingte Pulsation der Herzwand oder durch den Akkommodationsvorgang am Auge, erfolgt eine Messung des aktuellen Abstandes a sowie eine fortlaufende Registrierung in Echtzeit aller Empfangssignale auf allen Einzelelementen zur Bestimmung des Krümmungsradius. Damit ist auch eine Bestimmung der Dynamik der Krümmung möglich.

Das beschriebene Verfahren liefert die Krümmungsradien von Grenzflächen mit großer Genauigkeit weitgehend unabhängig von vorgelagerten Blenden oder vorliegenden Kappen anstelle von Vollkörpern. Teilweise lässt sich mittels des gleichen Ultraschallwandlers bei anderer Betriebsweise zusätzlich die Blendengröße bzw. Kappenhöhe ermitteln. Mit der Erfindung ist es möglich, den Krümmungsradius r mit einer Genauigkeit von unter 5% zu bestimmen.

Die angegebenen Anwendungsgebiete werden nachfolgend detailliert angegeben:

-    Durch die Bestimmung krümmungsbedingter Signalformänderungen und Änderungen der Signalhöhe ist bei der zerstörungsfreien Prüfung und in der Ultraschalldiagnostik eine Verbesserung der Bestimmung von Ort und Größe der Grenzfläche - zum Beispiel Materialfehler, Artefakte, Organgrenzen, umschriebene krankhafte Gewebsveränderungen - möglich.

-    Da sowohl gekrümmte Grenzflächen als auch Phasenübergänge Signalformänderungen - Signalhöhe, Phase, Länge, Größenverhältnisse der einzelnen Peaks - hervorrufen, führt die Bestimmung der Krümmungsradien zu genaueren Aussagen über den Phasenübergang an der akustisch reflektierenden Grenzfläche. So lassen sich gekrümmte Einschlüsse von Hohlräumen oder Delaminationen unterscheiden.

-    Außerdem kann nicht nur zwischen Einschluss und Hohlraum unterschieden werden, sondern es sind auch Aussagen über das Material des Einschlusses möglich. Da die registrierte Signalform vom Sendesignal, vom Abstand zwischen Sender/Empfänger und der Grenzfläche sowie vom Krümmungsradius und dem Impedanzsprung abhängt, können nach der Bestimmung des Krümmungsradius aus der Schaltdruckverteilung und aus der Signalform am Empfänger auch Informationen über den Impedanzunterschied erhalten werden. Solche Informationen können auch zur Gewebecharaktensierung/-differenzierung genutzt werden.

Die Erfindung wird anhand mehrerer Ausführungsbeispiele mittels mehrerer Zeichnungen erläutert:

Es zeigen:

Fig. 1    schematische Darstellungen der erfindungsgemäßen Anordnung zur Be- stimmung der Kombination von Krümmungsradien und Abständen an akus- tischen Grenzflächen in einem Messobjekt mit einem flüssigen und einem soliden Medium mittels eines Ultraschallwandlers mit sechs Schallwandler- elementen, wobei Fig. 1a die Anordnung mit ihren wesentlichen Bestandteilen und Fig. 1b eine vergrößerte Darstellung der Anordnung im Bereich Messobjekt und Ultraschallwandler zeigen,

Fig. 2    eine schematische Darstellung eines Kreisring-Ultraschallwandlers mit sechs ringförmigen Elementen, wobei ein Element zusätzlich in Segmente unterteilt ist, in Draufsicht auf die Schallfläche,

Fig. 3    schematische Darstellungen von geometrischen Betrachtungen der Refle- xion an Grenzflächen, wobei Fig. 3a eine ebene Grenzfläche, Fig. 3b eine konvex gekrümmte Grenzfläche bei gleicher Fokussierung wie in Fig. 3a und Fig. 3c eine konvex gekrümmte Grenzfläche bei einer Fokussierung im Krümmungsmktelpunkt zeigen,

Fig. 4    Darstellungen der Schalldruckverteilung auf dem empfangenden Ultraschallwandler, wobei Fig. 4a eine ebene Grenzfläche nach Fig.3a,

Fig. 4b eine konvex gekrümmte Grenzfläche nach Fig. 3b und
Fig. 4c eine Schallleistungsskala
darstellen,

Fig. 5    Darstellungen der Schalldruckverteilung auf der Gesamtfläche des Ultra- schallwandlers in einem Abstand von a=10mm zwischen der Grenzfläche und dem Empfänger bei unterschiedlichen Fokussierungen und unter- schiedlichen Krümmungsradien $r_1$= 8mm und $r_2$=10mm der Grenzflächen, wobei
Fig. 5a eine Fokussierung auf einen Fokusabstand $f_0$ von 10mm,
Fig. 5b eine Fokussierung auf einen Fokusabstand $f_1$ von 15mm und
Fig. 5c eine Fokussierung auf einen Fokusabstand $f_2$ von 20mm, zeigen,
wobei eine Normierung des Schalldrucks an jedem Fokusabstand $f_0,f_1,f_2$ auf den Schalldruck der zusammengefassten Elemente des Ultraschall- wandlers vorhanden ist,

Fig. 6    eine Darstellung der ermittelten Schalldruckverteilung mit Kurven $V=V(f_i)$ auf dem Empfänger zur Schnitt- punktermidung des von einer konvexen Kugelgrenzfläche mit einem ersten Krümmungsradius $r_1$=8mm reflektierten Signals auf sechs Elementen E1 bis E6 bei einer schrittweisen Fokussie- rung bei einem Abstand a=10mm nach Fig. 5, wobei eine Normierung an jedem Fokusabstand $f_i$ auf den Schalldruck der zusammengefassten Ele- mente des Ultraschallwandlers vorhanden ist,

Fig. 7    eine Darstellung der ermittelten Schalldruckverteilung mit Kurven $V=V(f_i)$ auf dem Empfänger zur Schnittpunktermittlung des von einer konvexen Kugelgrenzfläche mit einem zweiten Krümmungsradius $r_2$=10mm reflektier- ten Signals auf sechs Elementen E1 bis E6 bei einer schrittweisen Fokus- sierung bei einem Abstand von a=10mm nach Fig. 5, wobei eine Normie- rung an jedem Fokusabstand $f_i$ auf den Schalldruck der zusammengefass- ten Elemente des Ultraschallwandlers vorhanden ist, und

Fig. 8    eine Darstellung der ermittelten Schalldruckverteilung mit Kurven $V=V(f_i)$ auf dem Empfänger zur Schnitt- punktermidung des von einer konvexen Kugelgrenzfiäche mit einem Krümmungsradius r=9mm reflektier- ten Signals auf sechs Elementen E1 bis E6 bei einer schrittweisen Fokussierung bei einem Abstand von a=10mm nach Fig. 5, wobei eine Normierung an je- dem Fokusabstand $f_i$ auf den Schalldruck der zusammengefassten Ele- mente des Ultraschallwandlers vorhanden ist.

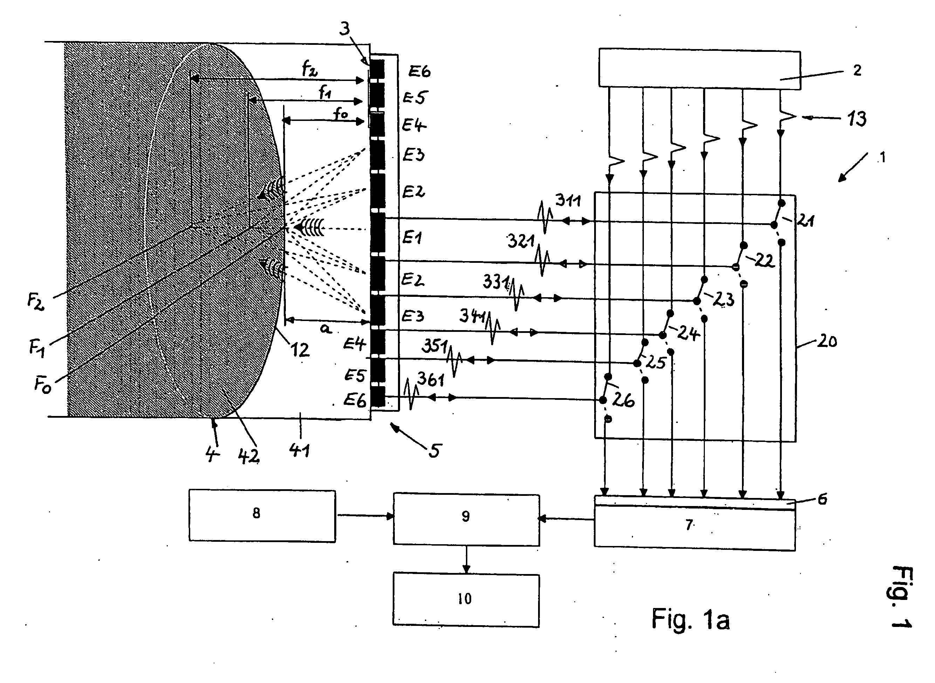

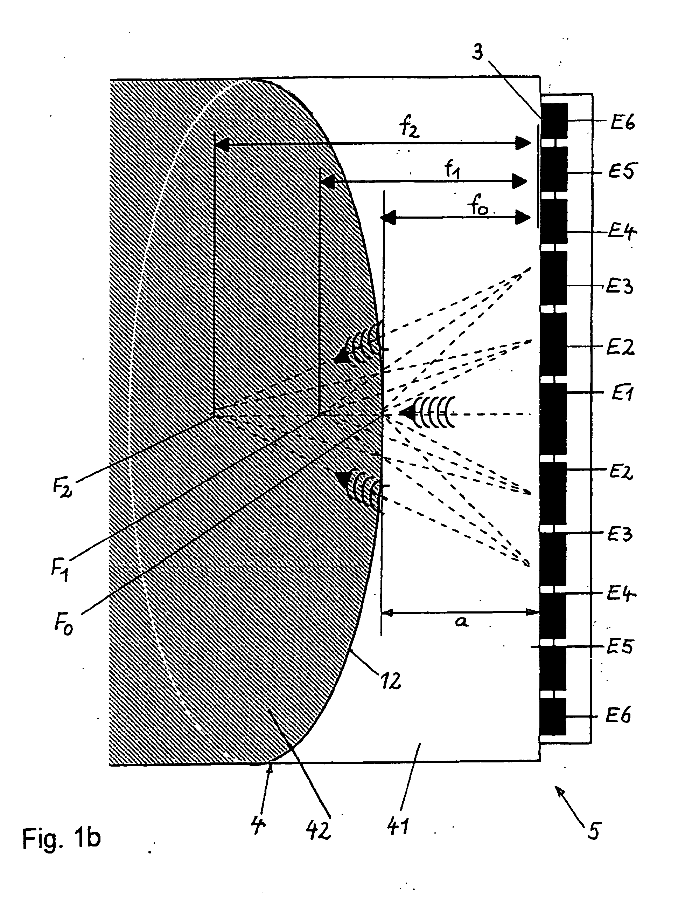

Im Folgenden werden die Fig. 1, Fig.1 a und 1b gemeinsam betrachtet

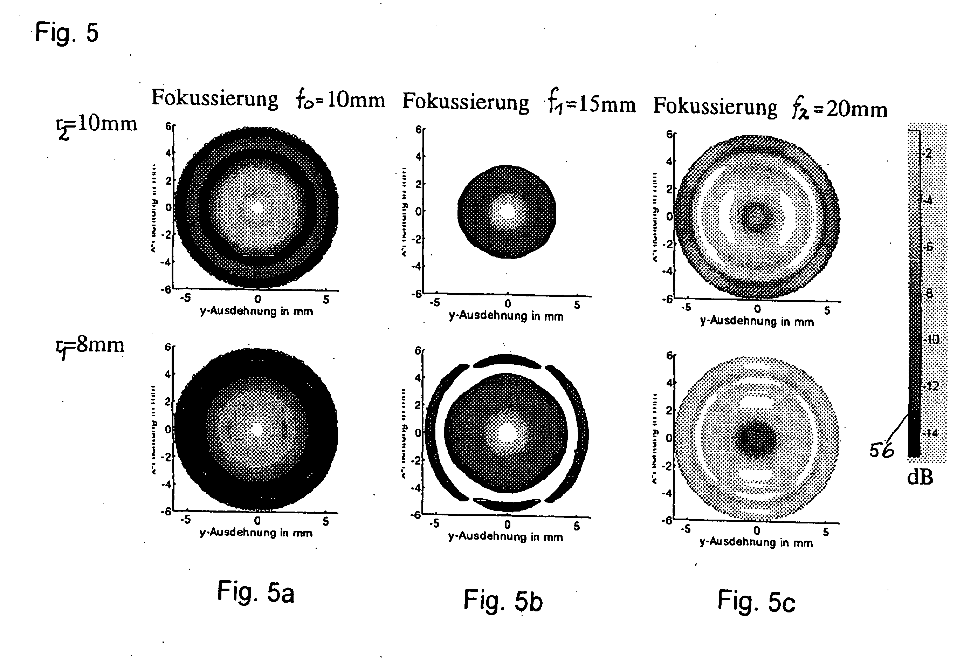

In Fig. 1 ist eine Anordnung 1 zur Bestimmung der Kombination von Krümmungsradius r und Abstand a der akustischen Grenzflächen 12 zwischen einem ersten Medium 51 und einem zweiten Medium 52 des Messobjektes 4 mittels Ultraschall mit Hilfe eines Ultraschallwandlers 3 mit sechs Schattwandler-Elementen E1,E2,E3,E4, E5,E6 dargestellt.
Die Anordnung 1 besteht aus
einem Pulsgenerator 2, der sechs gegeneinander zeitverzögerte Anregungssignale 13 erzeugt,
einer Sende-Empfangs-Weiche 20,
dem Ultraschallwandler 3 mit den sechs Elementen E1,E2,E3,E4,E5,E6, der die Signale 13 vom Pulsgenerator 2 über die Sende-Empfangs-Weiche 20 erhält und dessen Elemente E1,E2,E3,E4,E5,E6 zeitverzögert angeregt werden und der von jedem Element E1,E2,E3,E4,E5,E6 aus eine zeitverzögerte Welle 311,321,331,341, 351,361 in das Messobjekt 4 in diesem Beispiel ins Wasser als erstes Medium 41 sendet und nach Umschaltung auf Empfang durch Schalter 21,22,23,24,25,26 in der Sende-Empfangs-Weiche 20 die von der Grenzfläche 12 reflektierten Uttraschattwelen separat an jedem Element E1,E2,E3,E4,E5,E6 empfängt, wobei zumindest durch den Ultraschallwandler 3 und durch die Einschallrichtung zum Messobjekt 4 eine Messanordnung 5 vorgegeben ist,
sechs Verstärkungseinheiten 6, die die empfangenen Signale vom Ultraschallwandler 3 über die Sende-Empfangs- Weiche 20 erhalten, und
einer Aufnahmeeinheit 7, die mindestens einen Analog-Digital-Wandler zur Analog-Digital-Wandlung der von der Sende- Empfangs-Weiche 20 weitergeleiteten Signale enthält,
einer der Aufnahmeeinheit 7 nachgeordneten Auswerteeinheit 9 und einer Ergebnis-Anzeigeeinheit 10, die der Auswerteeinheit 9 nachgeordnet ist
Erfindungsgemäß ist der Ultraschallwandler 3 derart ausgebildet, dass er an das Messobjekt 4, an die Messanordnung 5 und an den Abstand a zur Grenzfläche 12 bezüglich der Nennfrequenz, Elementgröße und Elementform angepasst ist und die je nach dem gewählten Zeitverzögerungsregime aus der Überlagerung der Wellenzüge 311,321,331,341,351,361 der Einzelelemente E1,E2,E3,E4,E5,E6 entstehenden Fokusse $F_1$ mit i=$_{0,1,2}$ schrittweise entlang der Axialen 40 des Ultraschallwandlers 3 auf einzelne Orte in Fokusabständen $f_i$ wie z.B. $f_0,f_1,f_2$ in Fig.5 verschiebt sowie auf und hinter der reflektierenden Grenzfläche 12 platziert,

ist eine Simulationseinheit 8 vorgesehen, mit deren Hilfe das reflektierte Schallfeld auf dem Ultraschallwandler 3 für unterschiedliche Krümmungsradien r der Grenzfläche 12 in Abhängigkeit des vorgesehenen Ultraschallwandler 3, der Impedanzwerte im Messobjekt 4 und des Abstandes a zwischen Ultraschallwandler 3 und Grenzfläche 12 für ca. 20 bis 40 verschiedene Fokusabstände $f_i$ u.a. für $f_0,f_1,f_2$ simuliert wird, für den Abstand a eine Schar von Kalibrierungskurven für verschiedene Krümmungsradien r in Form von Schalldruckverteilungskurven und die Signalform jeweils als Funktion der Fokusabstände $f_i$ geliefert wird, wobei die Simulationseinheit 8 an die Auswerteeinheit 9 angeschlossen ist,

wobei die Aufnahmeeinheit 7 neben dem aus der Laufzeit bestimmten Abstand a der Grenzfläche 12 vom Ultraschallwandler 3 für den jeweiligen Fokusabstand $f_i$ z.B. $f_0,f_1,f_2$ das reflektierte Signal auf den einzelnen Elementen E1,E2,E3,E4,E5,E6 bestimmt, woraus der zeitliche Verlauf des Schalldrucks und daraus die Schalldruckamplitude auf jedem Element E1,E2,E3,E4,E5,E6 und für zusammengefasste Elemente, die Schalldruckverteilungsanteile auf die einzelnen Elemente E1,E2,E3,E4,E5,E6 für jeden Fokus $F_i$ z.B. $F_0,F_1,F_2$ sowie Signalformänderungen ermittelt werden, wobei die Schalldruckverteilungskurven als Funktion der Fokusabstände $f_i$ erstellt werden, und

wobei die durch Messung ermittelten Schalldruckverteilungskurven mit der Schar der Kalibrierungskurven für den ermittelten Abstand a zwischen der Grenzfläche 12 und dem Ultraschallwandler 3 verglichen werden, wobei die Auswerteeinheit 9 die Lage der Schnittpunkte S zwischen den Schalldruckverteilungskurven der Einzelelemente E1,E2,E3,E4,E5,E6 oder zusammengefasster Elemente bestimmt und mit den Kalibrierungskurven vergleicht und die zwei Scharen von Kalibrierungskurven auswählt, bei denen die Schnittpunkte S der Kurven der gleichen Elemente E1,E2,E3,E4,E5,E6 oberhalb und unterhalb der Schnittpunkte S in den gemessenen Schalldruckverteilungskurven liegen, und durch Interpolation aus der Schnittpunktlage den Krümmungsradius r bestimmt,

und wobei der bestimmte Krümmungsradius r in der Ergebnis-Anzeigeeinheit 10 angegeben wird.

Für das Messobjekt 4 im Ausführungsbeispiel, bestehend aus zwei Schichten, einem Wassermedium 41 als erstem Medium und Plexiglas 42 als zweitem Medium, mit einer sphärischen soliden Grenzfläche12 bei einem eingestellten Abstand a=10mm zwischen dem Ultraschallwandler 3 und der Grenzfläche 12 erfolgt eine Auswahl eines ebenen Ringschallwandlers 3 mit sechs Elementen E1,E2,E3,E4,E5,E6, der bezüglich der Grenzfläche 12 justiert wird. In den Fig. 1a, 1b ist der ebene Ringschallwandler 3 im Längsschnitt dargestellt.

Der zu bestimmende Krümmungsradius r wird der sphärisch gekrümmten Grenzflächen 12 zugeordnet

Die Auswahl des Ultraschallwandlers 3 wird mit Hilfe der Simulationseinheit 8 in Abhängigkeit vom Messobjekt 4, von der Messanordnung 5 und von der darin zu untersuchenden Grenzfläche 12 durchgeführt

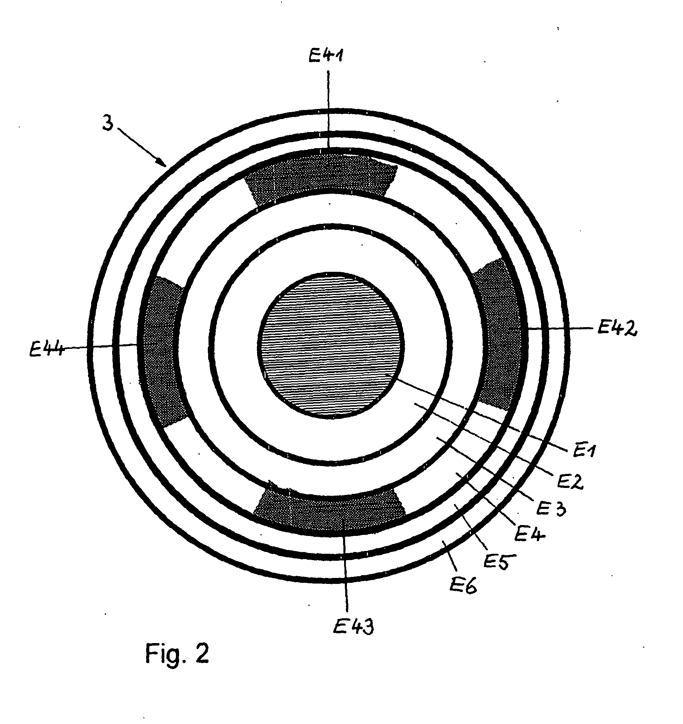

In Fig. 2 besitzen die einzelnen Elemente Ek=E1,E2,E3,E4,E5,E6 des Ultraschallwandlers 3 eine ringförmige Struktur, deren Form, Größe und zueinander vorgegebenen Abstände derart festgelegt sind.

Der Ultraschallwandler 3 weist in Abhängigkeit des Abstandes a zwischen Grenzfläche 12 und Ultraschallwandler 3 und des vorgegebenen Fokussierungs-Bereichs von ca. $f_i$=7mm bis 30mm eine ebene Fläche auf.

Bei gleichen Fokussierungsbedingungen im Ultraschallwandler 3 können zur Verminderung der Ringzahl Ringe gleicher Fläche, wie in Fig. 2 gezeigt ist, ausgebildet sein.

Im Ultraschallwandler 3 sind für den Empfang der reflektierten Schallwellen die Ringgrößen der einzelnen sechs Elemente El,E2,E3,E4,E5,E6 oder die durch Summation über einzelne nebeneinander liegender Elemente entstehenden Ringgrößen derart festgelegt, dass die registrierten Schalldruckunterschiede zwischen den einzelnen Ringen möglichst groß sind.

Im Ultraschallwandler 3 kann z.B., wie in Fig. 2 gezeigt ist, das ringförmige Element E4 aus den Elementen E1,E2,E3,E4,E5,E6 in Segmente E41,E42,E43,E44 unterteilt sein, um eine Schräglage der Grenzfläche für eine nachfolgende Justierung zu erkennen, um sphärische, torische und zylindrische Krümmung der Grenzfläche 12 zu unterscheiden sowie bei Segmentierung mehrerer Elemente E1,E2,E3,E4,E5,E6 und genügend großen Signalen eine Bestimmung von zwei Krümmungsradien der Hauptachsen zu erreichen.

Der Ultraschallwandler 3 kann sowohl mit einem üblichen breitbandigen Signal als auch mit einem schmalbandigen Signal zur Bestimmung der Schalldruckverteilung auf den einzelnen Elementen E1,E2,E3,E4,E5,E6 betreibbar sein.

Die Simulationseinheit 8 kann Kalibrierungskurven in Abhängigkeit vom Krümmungsradius r der Grenzfläche 12, vom Abstand a zwischen dem Ultraschallwandler 3 und der Grenzfläche 12, von der Elementegeometrie des Ultraschallwandlers 3 und von Impedanzunterschieden im Messobjekt 4 bereitstellen.

Die Aufnahmeeinheit 7 in Fig. 1 kann die Schalldruckanteile auf den ringförmigen Einzelelementen E1,E2,E3,E4,E5,E6 im Vergleich zum Gesamtschalldruck für den ganzen Ultraschallwandler 3 als Funktion der Fokusabstände $f_i$ bestimmen, wobei eine Normierung auf den Schalldruck der zusammengefassten Elemente des Ultraschallwandlers im jeweiligen Fokusabstand $f_i$ und eine Aufnahme der Schalldruckverteilungskurven erfolgt,

wobei durch Bestimmung der Verschiebung der Schnittpunkte S der Schalldruckverteilungskurven der Einzelelemente E1,E2,E3,E4,E5,E6 oder der zusammengefassten Elemente durch Vergleich mit den Kalibrierungskurven der Krümmungsradius r bestimmt wird.

Durch eine Normierung des Schalldruckes der Einzelelemente E1,E2,E3,E4,E5,E6 auf den Schalldruck der Gesamtfläche E1+E2+E3+E4+E5+E6 des Ultraschallwandlers 3 für den jeweiligen Fokus $F_i$ wird die Wirkung der Dämpfungseffekte beseitigt

Die Aufnahmeeinheit 7 nimmt außerdem den zeitlichen Verlauf des Signals für jedes Element E1,E2,E3,E4,E5,E6 auf, so dass Phasenänderungen des Signals und die Signalformänderung ausgewertet werden können.

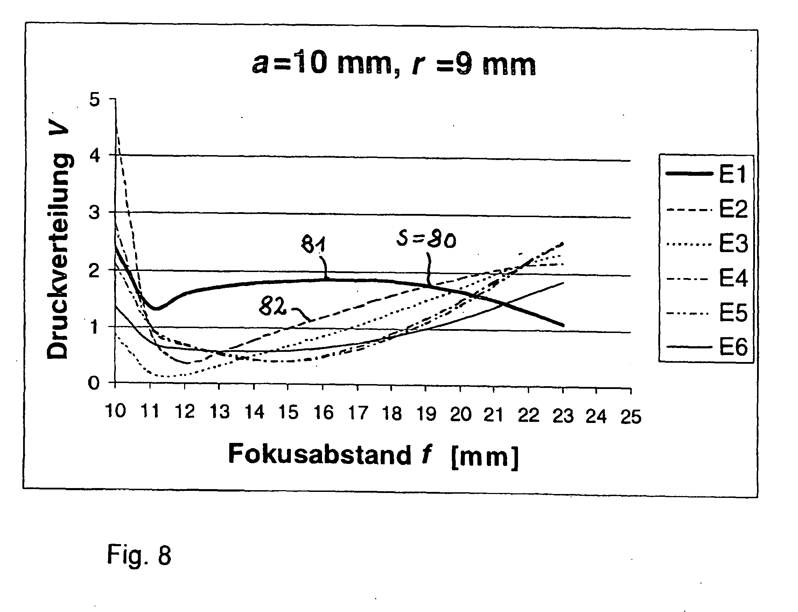

Das Verfahren zur Bestimmung der Kombination des Krümmungsradius r und des Abstandes a an der akustischen Grenzfläche 12 im Messobjekt 4, wobei sich die konvexe Grenzfläche 12 zwischen dem flüssigen ersten Medium 41 und dem soliden zweiten Medium 42 befindet, wird mittels Ultraschall mit der vorgenannten Anordnung 1 durchgeführt, unter Betrachtung der Fig. 1,5,6,7,8.

Erfindungsgemäß werden folgende Schritte durchgeführt:

- Simulation des reflektierten Schallfeldes auf dem Ultraschallwandler 3 für unterschiedliche Krümmungsradien $r_j$ der Grenzfläche 12 in Abhängigkeit des vorgesehenen Ultraschallwandlers 3, des zweischichtigen Messobjektes, der Messanordnung 5, der Impedanzen von Medium 41 und 42 und des Abstandes a zwischen Ultraschallwandler 3 und Grenzfläche 12 für die verschiedenen Fokusabstände im Bereich von $f_i$=7mm bis ca. 30mm in Schritten von 1 mm mit Hilfe der Simulationseinheit 8 und Erzeugung einer Schar von Kalibrierungskurven für verschiedene Krümmungsradien $r_j$ von $r_1$=8mm bis $r_2$=10mm in. Form von Schalldruckverteilungskurven und der Signalformen jeweils als Funktion der Fokusabstände $f_i$.

- Ermittlung des Abstandes a zwischen Ultraschallwandler 3 und Grenzfläche 12 aus der Laufzeit des empfangenen Signals durch die Auswerteeinheit 9 und Auswahl der Kalibrierungskurven und

- schrittweise Verschiebung der Fokusse $F_i$ entlang der Axialen 40 des Ultraschallwandlers 3 auf einzelne Orte in Fokusabständen im Bereich von $f_i$=7mm bis ca. 30mm in Schritten von 1mm auf und hinter der zu untersuchenden Grenzfläche 12 durch zeitverzögerte Überlagerung der Wellenzüge der Einzelelemente E1,EZ,E3,E4,E5,E6 und Messung der für den jeweiligen Fokusabstand $f_i$ reflektierten Schallwelle auf den einzelnen Elementen E1,E2,E3,E4,E5,E6 durch die Aufnahmeeinheit 7, woraus die Schalldruckamplihrde für jedes Element E1,E2,E3,E4,E5,E6 und für zusammengefasste Elemente, die Schalldruckverteh lungsanteile auf die einzelnen Elemente E1,E2,E3,E4,E5,E6 für jeden Fokusabstand $f_i$ sowie Signalformänderungen ermittelt werden, wobei die Schalldruckverteilungskurven als Funktion der Fokusabstände $f_i$ erstellt werden,

- Vergleich der durch Messung ermittelten Schalldruckverteilungskurven mit der Schar der Kalibrierungskurven für den ermittelten Abstand a und Bestimmung des Krümmungsradius r der Grenzfläche 12, wobei die Auswerteeinheit 9 die Lage der Schnittpunkte S zwischen den Schalldruckverteilungskurven der Einzelelemente E1,E2,E3,E4,E5,E6 oder zusammengefasster Elemente bestimmt und mit den Kalibrierungskurven vergleicht und den zwei Scharen von Kalibrierungskurven zuordnet, bei denen die Schnittpunkte S der Kurven der gleichen Elemente E1,E2,E3,E4,E5,E6 oberhalb und unterhalb der Schnittpunkte in den gemessenen Schalldruckverteilungskurven liegen, und durch Interpolation aus der Schnittpunktlage den Krümmungsradius r bestimmt, und

- Ausgabe des bestimmten Krümmungsradius r in der Ergebnis-Anzeigeeinheit 10.

Die Auswahl des Ultraschallwandlers 3 mit den angepassten Elementgrößen und Elementformen für die jeweiligen Parameter des Messobjektes 4 werden vor Beginn der Krümmungsradiusbestimmung durchgeführt. Der Ultraschallwandler 3 kann dabei mit Hilfe der Simulationseinheit 8 in Abhängigkeit des Messobjektes 4 festgelegt werden.

Der Ultraschallwandler 3 kann sowohl mit einem üblichen breitbandigen Signal als auch mit einem schmalbandigen Signal zur Bestimmung der Schalldruckverteilung auf den einzelnen Elementen E1,E2,E3,E4,E5,E6 betrieben werden.

Von der Simulationseinheit 8 werden Scharen von Kalibrierungskurven in Abhängigkeit vom Krümmungsradius r der Grenzfläche 12, von dem Abstand a zwischen dem Schallwandler 3 und von der zu untersuchenden Grenzfläche 12, von der Elementegeometrie des Ultraschallwandlers 3 und vom Messobjekt 4 bereitgestellt.

In der Aufnahmeeinheit 7 werden die Schalldruckanteile auf den Einzelelementen E1,E2,E3,E4,E5,E6 im Vergleich zum Gesamtschalldruck für den ganzen Ultraschallwandler 3 als Funktion der Fokusse $F_i$ in den Fokusabständen $f_i$ bestimmt, wobei eine Normierung auf den Schalldruck der zusammengefassten Elemente des Ultraschallwandlers 3 im jeweiligen Fokusabstand $f_i$ und eine Aufnahme der Schalldruckverteilungskurven erfolgen, wobei durch Bestimmung der Verschiebung der Schnittpunkte S der Schalldruckverteilungskurven der Einzelelemente E1,E2,E3,E4,E5,E6 oder der zusammengefassten Elemente durch Vergleich mit den Kalibrierungskurven der Krümmungsradius r bestimmt wird. Bei einem festgelegten Abstand a werden nur die dem Abstand a zugeordneten Kalibrierungskurven für unterschiedliche Krümmungsradien $r_j$ in die Bestimmung des wirklichen Krommungsradius r einbezogen.

Die Aufnahmeeinheit 7 nimmt außerdem den zeitlichen Verlauf des Signals für jedes Element E1,E2,E3,E4,E5,E6 auf, so dass außerdem Phasenänderungen des Signals und die Signalformänderung ausgewertet werden können.

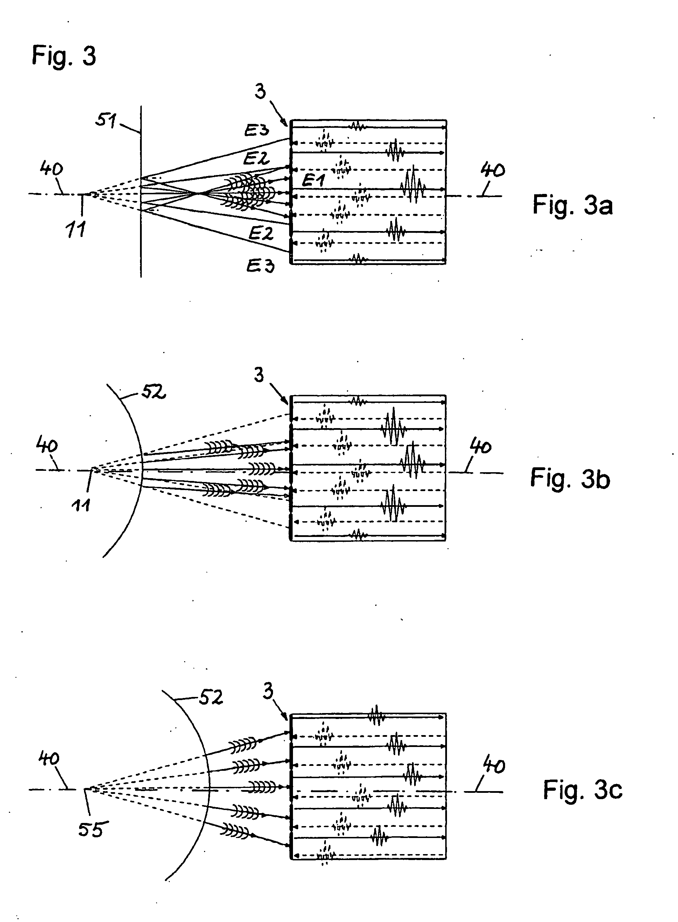

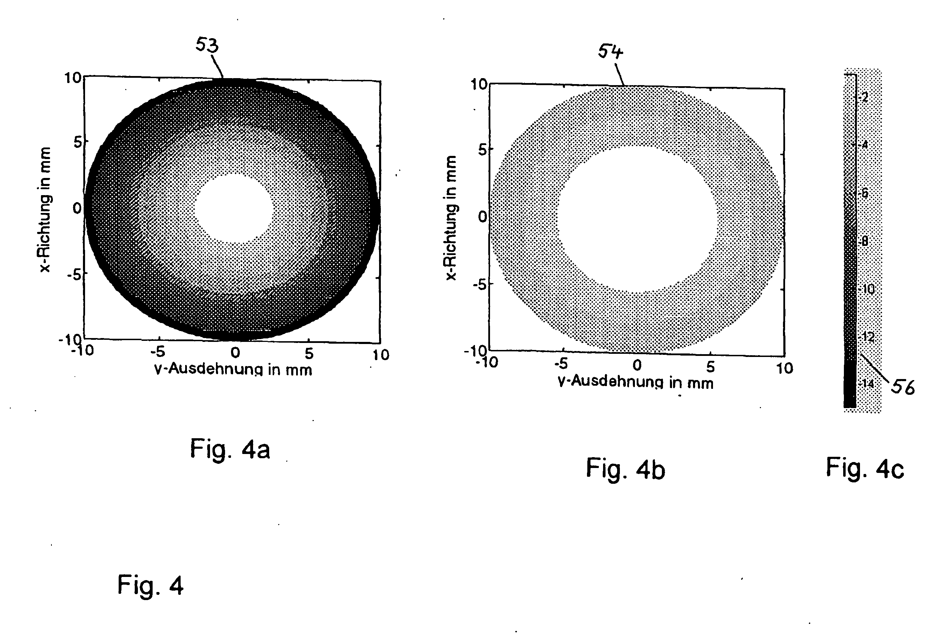

Die Krümmung einer Grenzfläche 12 spiegelt sich in der Schalldruckverteilung auf dem Ultraschallwandler 3 wider, was die geometrischen Überlegungen in Fig. 3,3a,3b,3c und die berechneten Schallfelder in Fig. 4,4a,4b zeigen.

In Fig. 3 sind schematische Darstellungen von geometrischen Betrachtungen der Reflexion an Grenzflächen mit einem Ultraschallwandler 3 mit drei Elementen E1,E2,E3, wobei Fig. 3a eine ebene Grenzfläche 51 mit dem Fokus 11 und Fig. 3b eine konvex gekrümmte Grenzfläche 52 mit gleicher Fokussierung im Fokus 11 wie in Fig. 3a sowie Fig. 3c eine konvex gekrümmte Grenzfläche 52 mit einer Fokussierung in den Krümmungsmitteipunkt 55 als Fokus zeigen. Der Vergleich von Fig.3a und 3b verdeutlicht, analog wie die berechneten Schallfelder in Fig. 4a und Fig. 4b, dass durch die konvexe Krümmung das Schallfeld divergenter wird, weil der Schalldruck auf Element E2 so groß wie der auf Element E1 wird. Durch schrittweise Fokussierung wird die Wirkung der gekrümmten Grenzfläche 52 verändert und kann zum Teil ausgeglichen werden, was Fig.3c zeigt. Dadurch verändert sich je nach Fokusabstand $f_i$ die Schalldruckverteilung auf dem Empfänger des Ultraschallwandlers 3.

In Fig.4 sind Darstellungen der Schalldruckverteilung V auf der Gesamtfläche des Ultraschallwandlers 3 - des Empfängers - gezeigt, wobei Fig. 4a das reflektierte Schallfeld 53 von der ebenen Grenzfläche 51 Fig. 4b die Schalldruckverteilung 54 für die konvex gekrümmte Grenzflächen 52 darstellt Fig. 4c zeigt eine für Fig. 4a und Fig. 4b gültige Schallleistungsskala 56 in Dezibel dB an. In beiden Fällen wird mit dem Sender nicht fokussiert. Das von der gekrümmten Grenzfläche 52 reflektierte Schallfeld in Fig.4b ist wesentlich divergenter als das von der ebenen Grenzfläche 51 reflektierte Schallfeld in Fig.4a. Somit spiegelt sich die Krümmung in der Schalldruckverteilung auf dem Empfänger des Ultraschallwandlers 3 wider.

In Fig. 5 sind Darstellungen der Schalldruckverteilung V auf die Gesamtfläche des Ultraschaltwandlers 3 in einem Abstand a mit a=10mm von der Grenzfläche 12 bei unterschiedlichen Fokussierungen und zwei Krümmungsradien $r_1$ = 8mm und $r_2$=10mm der Grenzflächen 12 angegeben, wobei Fig. 5a eine Fokussierung auf einen Fokusabstand von $f_0$=10mm, Fig. 5b eine Fokussierung auf einen Fokusabstand von $f_1$ =15mm und Fig. 5c eine Fokussierung auf einen Fokusabstand von $f_2$ =20mm zeigen. Die dargestellten Schalldruckverteilungen in den Fokusabständen $f_0,f_1,f_2$ sind charakteristische Bilder im Berechnungsbereich von $f_i$=7mm bis ca. 30mm, wobei sich der Fokus $F_0$ mit a = $f_0$ auf und die Fokusse $F_1,F_2$ mit a < $f_1,f_2$ hinter der Grenzfläche 12 in Bezug auf Fig. 1 befinden.

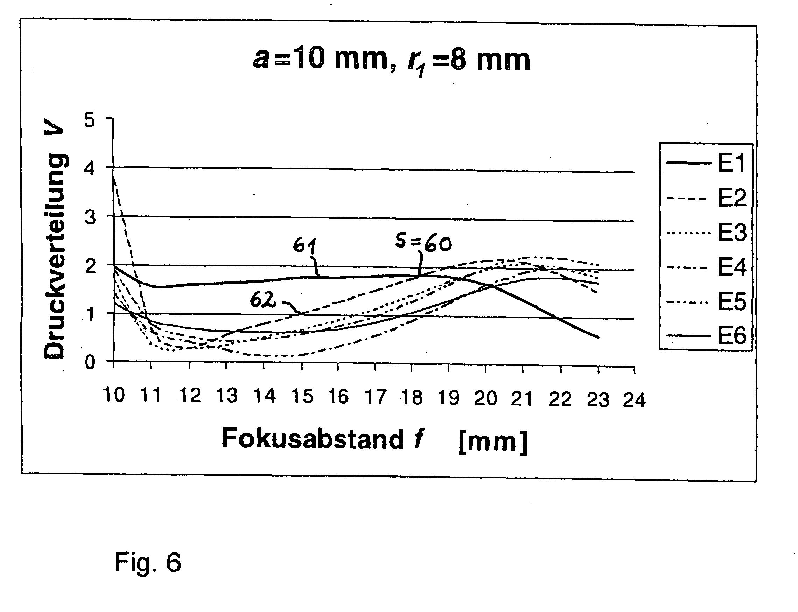

Die Fig. 6,7,8 zeigen für einen Grenzflächenabstand a mit a=10mm Schalldruckverteilungen V für drei verschiedene Grenzflächenkrümmungsradien $r_1$ = 8mm, r=9mm und $r_2$= 10mm in Abhängigkeit von den Fokusabständen $f_i$ in dem Ausschnitt $f_0$=10mm bis $f_i$=23mm, in dem sich die Schnittpunkte der Kurven befinden, wobei sich in diesem Fall alle Fokuspunkte auf bzw. hinter der Grenzfläche 12 in Bezug auf Fig. 1 befinden.

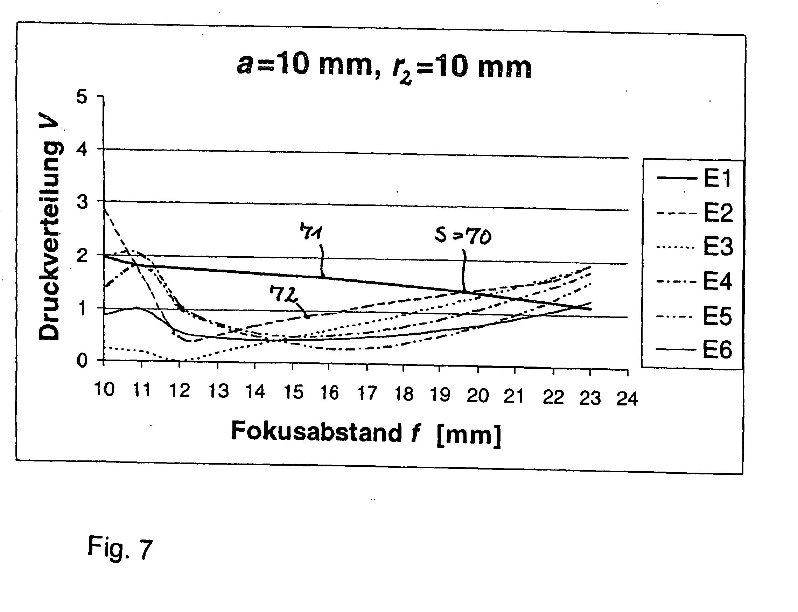

Die Fig. 6,7 zeigen beispielhaft zwei mit Hilfe von Schallfeldberechnungen ermittelte Kalibrierungskurven - Druckverteilungen V=V($f_i$) in Abhängigkeit von den Fokusabständen $f_i$ auf den einzelnen Elementen E1,E2,E3,E4,E5,E8 bei sechs Elementen für den Ultraschallwandler 3 für eine gekrümmte Grenzfläche 12 mit den Krümmungsradien $r_1$=8mm und $r_2$=10mm.

Die Kalibrierungskurven werden mit Hilfe von harmonischen Schallfeldberechnungen, die in der Druckschrift: Plane arrays - Fundamental investigations for correct steering by means of sound field calculations, Wave Motion, 44, 2007, S. 248-261 beschrieben sind, ermittelt wobei im Ausführungsbeispiel die Berechnungen für einen Abstand a=10mm zwischen Sender/Empfänger 3 und Grenzflächen 12 und für den Schallwandler 3, der für die Messungen eingesetzt wird, durchgeführt werden. Der reflektierte Schalldruck p wird dabei für jeden Fokus $F_1$ mit den Fokusabständen $f_i$ im Bereich von $f_i$=7mm bis 30mm durch phasenrichtige Überlagerung der Schallfelder der einzelnen Schallkopfelemente in Abhängigkeit des entsprechend der Fokussierung gewählten Verzögerungsregimes berechnet.

Für jeden Fokus $F_i$ wird entsprechend Gleichung (1) die Schalldruckamplitude für die Einzelelemente Ek ermittelt und auf die Amplitude des Schalldrucks der zusammengefassten Elemente des Ultraschaltwandlers 3 normiert, wobei Gleichung (I) mit

$$P_k(f_i) = \left| \frac{\underline{p}_k(f_i)}{\sum_{k=1}^{N} \underline{p}_k(f_i)} \right| \qquad \text{. (I)}$$

gegeben ist,
wobei k - Elementnummer, N - Anzahl der Elemente Ek, $f_i$ - Fokusabstand, $\underline{p}_k(f_i)$

der Schalldruck auf dem Element Ek für die Fokussierung $F_i$ und $\sum_{k=1}^{N} \underline{p}_k(f_i)$ der Schalldruck der zusammengefassten Elemente des Ultraschallwandlers 3 am jeweiligen Fokus $F_i$ sind.

Wird für alle Elemente die normierte Schalldruckamplitude in Abhängigkeit vom Fokusabstand $f_i$ aufgetragen, ergibt sich für eine Grenzfläche 12 mit den Krümmungsradius $r_1$=8mm die Kurvenschar in Fig.6 und für den Krümmung-

sradius $r_2$=10mm die Kurvenschar in Fig.7. Die so berechneten Kurvenscharen werden als Kalibrienrngskurven eingesetzt.

Die Bestimmung der Kalibrierungskurven durch Berechnung des harmonischen Schallfeldes ist korrekt, obwohl bei den Messungen transiente Signale Verwendung finden, wie in der Druckschrift Kühnicke, E.: Optimization of ultrasound broadband transducers for complex testing problems by means of transient and time harmonic sound fields, 9th European Conference on NDT, September, 2006, DGZfP Proceedings BB 1003-CD beschrieben ist. Es wird darin gezeigt dass das harmonische Schallfeld bei der jeweiligen Mittenfrequenz das transiente Schallfeld gut widerspiegelt.

Bei einem konstanten Krümmungsradius r, wie in der Kurvenschar in Fig.6 oder Fig.7 gezeigt, verschiebt sich mit abnehmender Fokussierung, das entspricht einem zunehmenden Fokusabstand $f_i$, das Maximum des Schalldrucks von dem inneren Element E1 zu den äußeren Elementen E5,E6 des Ultraschallwandlers 3, und es kommt durch die Änderung der Schalldruckverteilung auf die einzelnen Elemente Ek zu Schnittpunkten S zwischen den Schalldruckverteilungskurven der einzelnen Elemente Ek. An diesen Schnittpunkten S liegt auf den zwei zugehörigen Elementen die gleiche Schalldruckamplitude vor.

Da das reflektierte Schallfeld mit kleiner werdendem Krümmungsradius r divergenter wird, ändert sich die Lage der Schnittpunkte S in Abhängigkeit des Krümmungsradius r der Grenzfläche 12.

Die Tabelle 1 gibt in den ersten zwei Zeilen für $r_1$=8mm und $r_2$=10 mm die aus den Fig.6 bzw. Fig.7 ermittelten Schnittpunkte S der Druckverteilungskurven auf die Elemente Ek in Abhängigkeit von den Fokusabständen $f_i$ an. Werden die Schnittpunkte S=60,70 der Kurven 61,62 und 71,72 der einzelnen Elemente E1,E2 für die Krümmungsradien $r_1$=8mm und $r_2$=10mm in Fig. 6 und Fig. 7 betrachtet, so ist erkennbar, dass diese für den kleineren Krümmungsradius $r_1$ dichter an der Grenzfläche 12 liegen.

Aus den durch Messungen ermittelten Kurven, hier zur Abschätzung der erreichbaren Genauigkeit durch Rechnung ermittelten Kurven, wird der Krümmungsradius r ermittelt. Ein Vergleich mit den Kalibrierungskurven 61,62 und 71,72 zeigt, dass der Schnittpunkt S=80 der Kurven 81,82 der Einzelelemente E1,E2 zwischen den Schnittpunkten S=60,70 für die Kalibrienrngskunren für die Krümmungsradien $r_1$=8mm und $r_2$=10mm liegen. d.h. der gesuchte Krümmungsradius r für die Grenzfläche 12 hat einen Wert zwischen $r_1$=8mm und $r_2$=10mm. Der genaue Wert wird durch Interpolation ermittelt. Dazu wird die Verschiebung der Schnittpunkte $X_{KatiMerung}$ für $r_1$=8mm und $r_2$=10mm bestimmt. Die Verschiebung entspricht einer Krümmungsradiusänderung $\triangle r_{Kalibrlerung}$ von 2mm. Für die gemessenen Kurven werden die Schnittpunkte S ermittelt (Tab.1 4.Zeile) und daraus die Verschiebung der Schnittpunkte $\triangle x_{Messung}$ bzgl. der Schar von Kalibrierungskurven für $r_1$=8mm bestimmt (Tab.1, Zeile 5). Entspnechend der Gleichung (11)

$$\Delta r_{Messwert} = \frac{\Delta r_{Kalibrierung}}{\Delta x_{Kalibrierung}} \Delta x_{Messwert} \qquad (II)$$

ist die Radiusänderung gegenüber $r_1$=8mm aus der Schnittpunktverschiebung zu ermitteln (Tab.1,6.Zeile). Daraus ergibt sich entsprechend Gleichung (111) der Krümmungsradius $r_{ermlttelt}$ der Grenzfläche 12 (Tab.1, 7.Zeile).

$$r_{ermittelt} = r_i + \Delta r_{Messwert} \qquad (III)$$

Der Krümmungsradius $r_{ermittelt}$ ist überbestimmt Aus der Mitteilung ergibt sich ein Krümmungsradius $r_{ermittelt}$ von $r_M$=9mm (Tab.,8.Zeile), was dem wirklich verwendeten Grenzflächen-Krümmungsradius für diese Beispielrechnung entspricht. Durch Verwendung eines Schattwandler-Elementefeldes mit gleichen Elementflächen, aber angepassten Zwischenräumen lässt sich die notwendige Elementzahl für den Sender bei einigen Anwendungen reduzieren. Damit nimmt aber die Genauigkeit der Krümmungsradiusbestimmung, wie Tab.1 zeigt, ab.

Tabelle 1:

| Schnittpunkte S | S(E2/E1) | S(E3/E1) | S(E4/E1) | S(E5/E1) | S(E6/EI) |
|---|---|---|---|---|---|
| Kalibrierungskurve $r_1$=8mm - Fig. 6 | 18,12 | 19,33 | 20,0 | 19,33 | 20,13 |
| Kalibrierungskurve $r_2$=10mm - Fig. 7 | 19,67 | 20,31 | 21,16 | 21,86 | 22,47 |
| Abstand Schnittpunkt $\triangle x_{Ka- fibrierung}$, der $\triangle r_{Kalibrierung}$=$r_2$-$r_1$=2mm entspricht | 1.55 | 0.98 | 1,16 | 2.53 | 2.34 |

(fortgesetzt)

| Schnittpunkte S | S(E2/E1) | S(E3/E1) | S(E4/E1) | S(E5/E1) | S(E6/EI) |
|---|---|---|---|---|---|
|  |  |  |  |  |  |
| Gemessene Schalldruck-verteilungskurven (Kurvenschar Fig. 8) | 19,2 | 19,79 | 20,34 | 20,5 | 21,26 |
| $\Delta x_{Messwert}$ zu $r_1$ | 1.08 | 0.46 | 0.34 | 1.17 | 1.13 |
| $$\Delta r_{Messwert} = \frac{\Delta r_{Kalibrierung}}{\Delta x_{Kalibrierung}} \Delta x_{Messwert}$$ | 1,39 | 0,94 | 0,59 | 0.92 | 0,97 |
| $$r_{ermittelt} = r_1 + \Delta r_{Messwert} \quad :$$ | 9.39 | 8,94 | 8,59 | 8.92 | 8,97 |
| $r_M - r_{gemittelt}$ | 8.96 |  |  |  |  |

Die Lage der Schnittpunkte S=60,70,80 ist unabhängig von der reflektierenden Flächengröße, die z.B. durch eine Blende eingeschränkt sein kann.

Zur Bestimmung des Krümmungsradius r sind, wie in den Fig. 6 bis 8 gezeigt ist die Kurven 61,62;71,72;81,82 von jeweils zwei Elementen **E1,E2** ausreichend. Durch die Bestimmung mehrerer Schnittpunkte S und daraus des Krümmungsradius $r_{ermittelt}$ mit anschießender Mittelung des Krümmungsradius $r_M$ lassen sich Fehler durch bauliche Mängel des Ultraschallwandlers 3 minimieren. Deshalb ist es zweckmäßig, weitere Kreisringe E3,E4,E5,E6 am Ultraschalmandler 3, die zur Fokussierung, also für den Sender, erforderlich sind, bei der Auswertung am Empfänger des Ultraschallwandlers 3 zu berücksichtigen.

Bezugszeichenliste

1 Anordnung
2 Pulsgenerator
20 Sende-Empfangs-Weiche
21 erster Schalter
22 zweiter Schalter
23 dritter Schalter
24 vierter Schalter
25 fünfter Schalter
26 sechster Schalter
3 Ultraschallwandler
311 Schallsendesignal
321 Schallaendesignal
331 Schallsendesignal
341 Schallsendesignal
351 Schallsendesignal
361 Schallsendesignal
4 Messobjekt
40 Axialachse
41 Erstes Medium
42 Zweites Medium
5 Messanordnung
6 Verstärkungseinheit
7 Aufnahmeeinheit
8 Simulierungseinheit
9 Auswerteeinheit
10 Ergebnis-Anzeigeeinheit

11    Fokus
12    Grenzfläche
13    Signale
51    ebene Grenzfläche
52    gekrümmte Grenzfläche
53    Schalldruckverteilung
54    Schalldruckverteilung
55    Krümmungsmittelpunkt
56    Schallleistungsskala
60    Kurvenschnittpunkt
61    Druckverteilungskurve
62    Druckverteilungskurve
70    Kurvenschnittpunkt
71    Druckverteilungskurve
72    Druckverteilungskurve
80    Kurvenschnittpunkt
81    Druckverteilungskurve

82    Druckverteilungskurve

E1    erstes Element
E2    zweites Element
E3    drittes Element
E4    viertes Element
E41    erstes Elementsegment
E42    zweites Elementsegment
E43    drittes Elementsegment
E44    viertes Elementsegment
E5    fünftes Element
E6    sechstes Element

$F_0$    erster Fokus
$F_1$    zweiter Fokus
$F_2$    dritter Fokus
$F_i$    i-ter Fokus

$f_0$    erster Fokusabstand
$f_1$    zweiter Fokusabstand
$f_2$    dritter Fokusabstand
$f_i$    i-ter Fokusabstand

$P_{-k}(Fi)$    Schalldruck
$r_1$    erster Krümmungsradius
$r_2$    zweiter Krümmungsradius
r    ermittelter Krümmungsradius

V    Druckverteilung
S    Schnittpunkt

**Patentansprüche**

1. Anordnung (1) zur Bestimmung der Kombination von Krümmungsradien (r) und Abständen (a) an akustischen Grenzflächen (12, 51, 52) in Messobjekten (4) mittels Ultraschall, bestehend aus

einem Pulsgenerator (2), der ausgestaltet ist, um n>2, mit n $\in$ N gegeneinander zeitverzögerte Anregungssignale (13) zu erzeugen,
einer Sende-Empfangs-Weiche (20),

einem Ultraschallwandler (3) mit n>2, mit $n \in N$ Elementen (E1, E2, E3, E4, E5, E6) ausgestaltet zum Senden und Empfangen von Schallwellen, und um die Signale (13) vom Pulsgenerator (2) über die Sende-Empfangs-Weiche (20) zu erhatten und dessen Elemente (E1, E2, E3, E4, E5, E6) ausgestaltet sind, um zeitverzögert angeregt zu werden und der weiter ausgestaltet ist, um von jedem Element (E1, E2, E3, E4, E5, E6) aus, eine zeitverzögerte Schallwelle (311, 321, 331, 341, 351, 361) in das Messobjekt (4) zu einer Grenzfläche (12, 51, 52) des Messobjekts zu senden und der weiter ausgestaltet ist, um nach Umschaltung auf Empfang durch einen Schalter (21, 22, 23, 24, 25, 26) in der Sende-Empfangs-Weiche (20) die von der Grenzfläche (12, 51, 52) reflektierten Schallwellen separat an jedem Element (E1, E2, E3, E4, E5, E6) zu empfangen, wobei zumindest durch den Ultraschallwandler (3) und durch die Einschallrichtung zum Messobjekt (4) eine Messanordnung (5) vorgegeben ist,

n>2, mit $n \in N$ Verstärkungseinheiten (6), die ausgestaltet sind, um die empfangenen Signale vom Ultraschallwandler (3) über die Sende-Empfangs-Weiche (20) zu erhalten,
einer Aufnahmeeinheit (7), die mindestens einen Analog-Digital-Wandler zur Analog-Digital-Wandlung der von der Sende-Empfangs-Weiche (20) über die Verstärkungseinheiten (6) weitergeleiteten Signale enthält,
einer der Aufnahmeeinheit (7) nachgeordneten Auswerteeinheit (9) und
einer Ergebnis-Anzeigeeinheit (10), die der Auswerteeinheit (9) nachgeordnet ist,
wobei der Ultraschallwandler (3) ausgestaltet ist, um die je nach einem gewählten Zeitverzögerungsregime aus der Überlagerung der Schallwellenzüge (311, 321, 331, 341, 351, 361) der Einzelelemente (E1, E2, E3, E4, E5, E6) entstehenden Fokusse ($F_i$ mit i=0,1,2,...,m) schrittweise entlang der Axialen (40) des Ultraschallwandlers (3) auf einzelne Orte in entsprechende Fokusabständen ($f_i$) zu verschieben und vor, auf und hinter der reflektierenden Grenzfläche (12, 51, 52) zu platzieren,
eine Simulationseinheit (8), die ausgestaltet ist, um das reflektierten Schallfeld auf dem Ultraschallwandler (3) für unterschiedliche Krümmungsradien ($r_j$) der Grenzfläche (12, 51, 52) und für unterschiedliche Abstände ($a_l$) zwischen Ultraschallwandler (3) und Grenzfläche (12, 51, 52) in Abhängigkeit des vorgesehenen Ultraschallwandlers (3), des Messobjekt (4) und der Messanordnung (5) für die verschiedenen Fokusse ($F_i$) zu simulieren, wobei für jeden Abstand ($a_l$) und für verschiedene Krümmungsradien ($r_j$) jeweils eine Schar von Kalibrierungskurven in Form von Schalldruckverteilungskurven als Funktion der Fokusabstände ($f_i$) und die Signalform geliefert wird, wobei die Simulationseinheit (8) an die Auswerteeinheit (9) angeschlossen ist,
die Aufnahmeeinheit (7) ausgestaltet ist, um neben dem aus der Laufzeit bestimmten Abstand (a) zwischen der Grenzfläche (12, 51, 52) und dem Ultraschainvandler (3) für alle verschiedenen Fokusse ($F_i$) die reflektierte Schallwelle auf den einzelnen Elementen (E1, E2, E3, E4, E5, E6) zu bestimmen und daraus für jedes Element (E1, E2, E3, E4, E5, E6) und/oder für zusammengefasste Elemente den zeitlichen Verlauf des Schalldruckes und daraus die Schall-Amplitude auf jedem Element (Ek) sowie Schalldruckverteilungsanteile auf den einzelnen Elementen (E1, E2, E3, E4, E5, E6) für jeden Fokus ($F_i$) sowie Signalformänderungen zu ermitteln und dabei die Schalldruckverteilungskurven als Funktion der Fokusabstände ($f_i$) zu erstellen, und
die Auswerteeinheit (9) ausgestaltet ist, um die durch Messung ermittelten Schalldruckverteilungskurven mit der Schar der Kalibrierungskurven für den ermittelten Abstand (a) zwischen Grenzfläche (12, 51, 52) und Ultraschallwandler (3) zu vergleichen, wobei die Auswerteeinheit (9) ausgestaltet ist, um die Lage der Schnittpunkte (S) zwischen den Schalldruckverteilungskurven der Einzelelemente (E1, E2, E3, E4, E5, E6) oder zusammengefasster Elemente zu bestimmen und mit den Kalibrierungskurven zu vergleichen und die zwei Scharen von Kalibrierungskurven auszuwählen, bei denen die Schnittpunkte (S) der Kurven der gleichen Elemente (E1, E2, E3, E4, E5, E6) oberhalb und unterhalb der Schnittpunkte (S) in den gemessenen Schalldruckverteilungskurven liegen und durch Interpolation aus der Schnittpunktlage den Krümmungsradius (r) zu bestimmen, und
die Ergebnis-Anzeigeeinheit (10) ausgebildet ist, um den bestimmten Krümmungsradius (r) anzugeben.

2. Anordnung nach Anspruch 1,
wobei die Simulationseinheit (8) derart ausgebildet ist, um den für die Bestimmung zutreffenden Ultraschallwandler (3) mit Hilfe der Simulationseinheit (8) in Abhängigkeit des Messobjektes (4), der darin enthaltenen Materialien, der Messanordnung (5), des Abstandes (a) der zu untersuchenden Grenzfläche (12, 51, 52) und der unterschiedlichen Krümmungsradien **($r_j$),** die voraussichtlich auftreten, auszuwählen.

3. Anordnung nach Anspruch 2,
wobei die einzelnen Elemente (E1, E2, E3, E4, E5, E6) des Ultraschallwandlers (3) eine ringförmige Struktur besitzen, deren Form, Größe und zueinander bezogenen Abstände derart festgelegt sind, dass eine schrittweise Fokussierung in ausreichenden Fokusabständen ($f_i$) vor, auf und hinter der Grenzfläche (12, 51, 52) möglich ist.

4. Anordnung nach Anspruch 1 bis 3,
   wobei der Ultraschallwandler (3) in Abhängigkeit des voraussichtlich auftretenden Abstandes (a) zwischen zu untersuchender Grenzfläche (12, 51, 52) und Ultraschallwandler (3) und der daraus resultierenden Fokusabstände ($f_i$) entweder eben ist oder auf einer Kalotte platzierte Elemente (E1, E2, E3, E4, E5, E6) aufweist.

5. Anordnung nach Anspruch 4,
   wobei der Ultraschallwandler (3) als Sender zur besseren Fokussierung als Einzelelemente (E1, E2, E3, E4, E5, E6) Ringe gleicher Fläche besitzt.

6. Anordnung nach Anspruch 4,
   wobei beim Ultraschallwandler (3) die Ringgrößen oder die durch Summation über einzelne nebeneinander liegende Elemente (E1, E2, E3, E4, E5, E6) entstehenden Ringgrößen derart festgelegt sind, dass Schalldruckunterschiede zwischen den einzelnen Ringen registrierbar sind.

7. Anordnung nach Anspruch 6,
   wobei der Ultraschallwandler (3) als Sender und Empfänger mit Hilfe der Simulationseinheit (8) eine Anpassung an die Messbedingungen erfährt, wobei die Parameter des Ultraschallwandlers (3) mit Hilfe der Ergebnisse der Simulationseinheit (8) in Bezug auf das Messobjekt (4) angepasst sind, wobei der Ultraschallwandler (3) derart ausgewählt oder gefertigt ist, dass im Sendebetrieb eine Fokussierung möglich ist und im Empfangsbetrieb Schalldruckverteilungen registrierbar sind.

8. Anordnung nach Anspruch 7,
   wobei im Ultraschallwandler (3) mindestens ein ringförmiges Element (E4) in Segmente (E41, E42, E43, E44) unterteilt ist, um eine Schräglage der Grenzfläche (12, 51, 52) für eine nachfolgende Justierung zu erkennen, um sphärische, torische und zylindrische Krümmungen der Grenzflächen (12, 51, 52) zu unterscheiden sowie bei genügend großen Signalen eine Bestimmung von zwei Krümmungsradien (r) der Hauptachsen zu erreichen.

9. Anordnung nach einem der vorhergehenden Ansprüche,
   wobei der Ultraschallwandler (3) sowohl mit einem breitbandigen Signal als auch mit einem schmalbandigen Signal zur Bestimmung der Schalldruckverteilung zwischen den einzelnen Elementen (E1, E2, E3, E4, E5, E6) in Form harmonischer Wellenzüge bestimmter Länge oder in Form kurzer Impulse betreibbar ist.

10. Anordnung nach Anspruch 1,
    wobei die Simulationseinheit (8) ausgebildet ist, um Kalibrierungskurven in Abhängigkeit vom Messobjekt (4), von der Messanordnung (5), vom Ultraschallwandler (3), von unterschiedlichen Krümmungsradien ($r_j$), die voraussichtlich auftreten, von verschiedenen Abständen ($a_i$) zwischen dem Ultraschallwandler (3) und der Grenzfläche (12, 51, 52) bereitzustellen.

11. Anordnung nach Anspruch 1,
    wobei die Aufnahmeeinheit (7) derart ausgebildet ist, dass diese die Schalldruckanteile auf den Einzelelementen (E1, E2, E3, E4, E5, E6) im Vergleich zum Gesamtschalldruck für den ganzen Ultraschallwandler (3) als Funktion des Fokusabstandes ($f_i$) bestimmen, wobei gleichzeitig eine Bestimmung des zeitlichen Verlaufs des Schalldrucks für jedes Einzelelement (E1, E2, E3, E4, E5, E6) im jeweiligen Fokus $(F_i)$, eine Normierung auf den Schalldruck der zusammengefassten Elemente des Ultraschallwandlers (3) im jeweiligen Fokusabstand $(f_i)$ und eine Aufnahme der Schalldruckverteilungskurven des Spitzenschalldrucks oder des Pulsintegrals des quadrierten Schalldrucks als Funktion des Fokusabstandes ($f_i$) erfolgt,
    worauf die Auswerteeinheit (9) derart ausgebildet ist, dass diese die Lage der Schnittpunkte (S) in den Schalldruckverteilungskurven der Einzelelemente (E1, E2, E3, E4, E5, E6) oder zusammengefasster Elemente bestimmt und mit den Kalibrierungskurven vergleicht und die zwei Kalibrierungskurven auswählt, bei denen die Schnittpunkte (S) der gleichen Elemente oberhalb und unterhalb der Schnittpunkte (S) in den gemessenen Schalldruckverteilungskurven liegen, und durch Interpolation aus der Schnittpunktlage den Krümmungsradius (r) bestimmt

12. Anordnung nach Anspruch 11,
    wobei die Aufnahmeeinheit (7) derart ausgebildet ist, dass diese den zeitlichen Verlauf des Signals für jedes Element (E1, E2, E3, E4, E5, E6) aufnimmt, so dass Phasenänderungen des Signals und die Signalformänderung auswertbar sind.

13. Verfahrens zur Bestimmung der Kombination von Krümmungsradien (r) und Abständen (a) an akustischen Grenzflächen (12, 51, 52) in Messobjekten (4) mittels Ultraschall mit der Anordnung nach Anspruch 1 bis 12, mit folgenden Schritten:

- Simulation des reflektierten Schallfeldes auf dem Ultraschallwandler (3) für unterschiedliche Krümmungsradien ($r_j$) der zu untersuchenden Grenzfläche (12, 51, 52) in Abhängigkeit des vorgesehenen Ultraschallwandlers (3), des Messobjektes (4), der Messanordnung (5), der Impedanzunterschiede an der Grenzfläche (12, 51, 52) und des Abstandes ($a_l$) zwischen Ultraschallwandler (3) und Grenzfläche (12, 51, 52) für die verschiedenen Fokusabstände **($f_i$)** mit Hilfe einer Simulationseinheit (8) und Erzeugung einer Schar von Kalibrierungskurven für verschiedene Krümmungsradien ($r_j$) in Form von Schalldruckverteilungskurven und der Signalformen jeweils als Funktion der Fokusabstände **($f_i$),**
- Ermittlung der Abstände ($a_i$) zwischen Ultraschallwandler (3) und Grenzfläche (12, 51, 52) aus der Laufzeit des Ultraschallsignals durch die Aufnahmeeinheit (7) und Auswahl der Kalibrierungskurven,
- schrittweise Verschiebung der Fokusse ($F_i$) entlang der Axialen (40) des Ultraschallwandlers (3) auf einzelne Orte in entsprechenden Fokusabständen ($f_i$) vor, auf und hinter die zu untersuchende Grenzfläche (12, 51, 52) durch zeitverzögerte Überlagerung der Wellenzüge der Einzelelemente (E1, E2, E3, E4, E5, E6) und Messung der für den jeweiligen Fokusabstand ($F_i$) reflektierten Schallwelle auf den einzelnen Elementen (E1, E2, E3, E4, E5, E6) durch die Aufnahmeeinheit (7), woraus die Schalldruckamplitude für jedes Element (E1, E2, E3, E4, E5, E6) und für zusammengefasste Elemente, die Schalldruckverteilungsanteile auf die einzelnen Elemente (E1, E2, E3, E4, E5, E6) für jeden Fokusabstand ($f_i$) sowie Signalformänderungen ermittelt werden, wobei die Schalldruckverteilungskurven als Funktion der Fokusabstände ($f_j$) erstellt werden, und
- Vergleich der durch Messung ermittelten Schalldruckverteilungskurven mit der Schar der Kalibrierungskurven für den ermittelten Abstand (a) und Bestimmung des Krümmungsradius (r) der Grenzfläche (12, 51, 52), wobei die Auswerteeinheit (9) die Lage der Schnittpunkte (S) zwischen den Schalldruckverteilungskurven der Einzelelemente (E1, E2, E3, E4, E5, E6) oder zusammengefasster Elemente bestimmt und mit den Kalibrierungskurven vergleicht und den zwei Scharen von Kalibrierungskurven zuordnet, bei denen die Schnittpunkte (S) der Kurven der gleichen Elemente oberhalb und unterhalb der Schnittpunkte (S) in den gemessenen Schalldruckverteilungskurven liegen und durch Interpolation aus der Schnittpunktlage den Krümmungsradius (r) bestimmt, und
- Ausgabe des bestimmten Krümmungsradius (r) in der Ergebnis-Anzeigeeinheit (10).

**14.** Verfahren nach Anspruch 13,
wobei die Auswahl des Ultraschallwandlers (3) mit Hilfe der Simulationseinheit (8) entsprechend den Parametern des Messobjekts (4) vor Beginn des Verfahrens zur Krümmungsradiusbestimmung durchgeführt wird.

**15.** verfahren nach Anspruch 13 oder 14,
wobei von der Simulationseinheit (8) Kalibrierungskurven in Abhängigkeit vom Krümmungsradius (r) der Grenzfläche (12, 51, 52), vom Abstand (a) zwischen dem Ultraschallwandler (3) und der zu untersuchenden Grenzfläche (12, 51, 52), von der Elementegeometrie des Ultraschallwandlers (3), von Messanordnung (5) und vom Messobjekt (4) als Funktion des Fokusabstandes **($f_i$)** bereitgestellt werden.

**16.** Verfahren nach einem der Ansprüche 13 bis 15,
wobei in der Aufnahmeeinheit (7) die Schalldruckanteile auf den Einzelelementen (E1, E2, E3, E4, E5, E6) im Vergleich zum Gesamtschalldruck für den ganzen Schallwandler (3) als Funktion des Fokusabstandes ($f_i$) bestimmt werden, wobei eine Normierung auf den Schalldruck der zusammengefassten Elemente des Ultraschainvandlers (3) im jeweiligen Fokusabstand ($f_i$) und eine Aufnahme der Schalldruckverteilungskurven erfolgen, wobei durch Bestimmung der Verschiebung der Schnittpunkte (S) der Schalldruckverteilungskurven der Einzelelemente (E1, E2, E3, E4, E5, E6) oder der zusammengefassten Elemente durch Vergleich mit den Kalibrierungskurven der Krümmungsradius (r) bestimmt wird.

**17.** Verfahren nach mindestens einem der vorhergehenden Ansprüche 13 bis 16,
wobei durch die Normierung des Schalldruckes der Einzelelemente (E1, E2, E3, E4, E5, E6) auf den Schalldruck der Gesamtfläche (E1+E2+E3+E4+E5+E6) des Ultraschallwandlers (3) für den jeweiligen Fokus ($F_i$) die Wirkung der Dämpfungseffekte minimiert wird.

**18.** Verfahren nach mindestens einem der vorhergehenden Ansprüche 13 bis 17, wobei die Aufnahmeeinheit (7) außerdem den zeitlichen Verlauf des Signals für jedes Element (E1, E2, E3, E4, E5, E6) aufnimmt, so dass Phasenänderungen des Signals und die Signalformänderung ausgewertet werden.

**19.** Verfahren nach mindestens einem der vorhergehenden Ansprüche 13 bis 18,
wobei die Bestimmung von Krümmungsradius (r) und die Form der Grenzfläche (12, 51, 52) und deren Abstand (a) aus dem Ultraschall-Echosignal bei Grenzflächen (12, 51, 52) durchgeführt werden, wobei Sender/Empfänger des Ultraschallwandlers (3) und Grenzfläche (12, 51, 52) zueinander konstant justiert und in Ruheposition bleiben, wobei die reflektierende Grenzfläche (12, 51, 52) vorgegeben eben sowie sphärisch, torisch und/oder zylindrisch gekrümmt ist, wobei die Krümmungsradiusinformation durch Variation der gesendeten Wellenfront und durch Diskretisierung des Schallfeldes am Empfänger des Ultraschallwandlers (3) gewonnen werden.

**20.** Verfahren nach mindestens einem der vorhergehenden Ansprüche 13 bis 18,
wobei durch eine gleichzeitige kombinierte Messung des Abstandes (a) und des Krümmungsradius (r) die Bestimmung des Krümmungsradius (r) der Grenzfläche (12, 51, 52) auch bei dynamischen Vorgängen - bei Bewegung der Grenzfläche (12, 51, 52) - durchgeführt wird, wobei die Krümmungsradiusinformation durch Variation der gesendeten Wellenfront und durch Diskretisierung des Schallfeldes am Empfänger des Ultraschallwandlers (3) gewonnen werden.

**21.** Verfahren nach mindestens einem der vorhergehenden Ansprüche 13 bis 20,
wobei die Bestimmung der Krümmung einer Grenzfläche, der weitere Grenzflächen vorgelagert sind, durchgeführt wird.

**22.** Verfahren nach mindestens einem der vorhergehenden Ansprüche 13 bis 21,
wobei eine zusätzliche Ultraschallwandlerbewegung zur Verfolgung von Blasen in Strömungs- und Düfusions-Prozessen flüssiger Medien und/oder zur Vergrößerung des Messbereiches, vorzugsweise bei Erfassung des Krümmungsverlaufes über einen Bereich des Vielfachen der Schallbündelbreite, durchgeführt wird.

**Claims**

**1.** Assembly (1) and method for determining the combination of curvature radii (r) and distances (a) to acoustic boundary areas (12, 51, 52) in test objects (4) with ultrasound, consisting of:

a pulse generator (2) designed to generate n>2, with $n \in$ $\epsilon$ N interlocking time-delayed excitation signals (13),
a duplexer (20),

an ultrasound transducer (3) with n>2, with n € $\epsilon$ N elements (E1, E2, E3, E4, E5, E6) designed for transmitting and receiving of sound waves, and to receive the signals (13) from the pulse generator via the duplexer (2) and the elements (E1, E2, E3, E4, E5, E6) of which are designed to be excited with a time delay and furthermore designed to allow transmitting a time-delayed sound wave (311, 321, 331, 341, 351) from each element (E1, E2, E3, E4, E5, E6) into the test object (4) to a boundary area (12, 51, 52) of the test object (4) and furthermore designed to receive, after switching to reception mode, by means of a switch (21, 22, 23, 24, 25) in the duplexer (20), the sound waves reflected by the boundary area (12, 51, 52) separately at each element (E1, E2, E3, E4, E5, E6), wherein a test setup (5) is predetermined at least by the ultrasound transducer (3) and by the direction of incidence to the test object (4),

n>2, with n € $\epsilon$ N amplifier units (6), designed to receive the received signals from the ultrasound transducer (3) via the duplexer (20),
a recording unit (7), containing at least one analog-digital transducer for analog-digital conversion of the signals transmitted by the duplexer (20) via the amplifier units (6),
an evaluation unit (9) arranged downstream of the recording unit (7), and
a result display unit (10) arranged downstream of the evaluation unit (9),
wherein the ultrasound transducer (3) is designed to shift the developing focuses ($F_i$ with i=0, 1, 2, ..., m), depending on the selected time delay regime from the overlap of sound wave trains (311, 321, 331, 341, 351) of the individual elements (E1, E2, E3, E4, E5, E6), incrementally along the axials (40) of the ultrasound transducer (3) to individual locations in corresponding focal distances ($f_i$), and to place them in front of, on and behind the reflecting boundary area (12, 51, 52),
a simulation unit (8) designed to simulate the reflected sound field on the ultrasound transducer (3) for different curvature radii ($r_i$) of the boundary area (12, 51, 52) and for different distances ($a_l$) between ultrasound transducer (3) and boundary area (12, 51, 52), depending on the specified ultrasound transducer (3), the test object (4) and the test setup (5) for the different focuses ($F_i$), wherein a band of calibration curves in form of sound pressure distribution curves is to be delivered for each distance ($a_i$) and for different curvature radii ($r_i$) as a function of the focal distances ($f_i$), wherein the simulation unit (8) is connected to the evaluation unit (9),

the recording unit (7) designed to determine, aside from the distance (a) between the boundary area (12, 51, 52) and the ultrasound transducer (3) for all different focuses ($F_i$), the reflected sound wave on the individual elements (E1, E2, E3, E4, E5, E6), to determine from that the time curve of the sound pressure for each element (E1, E2, E3, E4, E5, E6) and/or for grouped elements and from that to determine the sound amplitude on each element ($E_k$) as well as the sound pressure distribution shares on the individual elements (E1, E2, E3, E4, E5, E6) for each focus ($F_i$) as well as signal form changes while generating the sound pressure distribution curves as a function of the focal distances ($f_i$), and

the evaluation unit (9) designed to compare the sound pressure distribution curves determined by measurement to the band of calibration curves for the determined distance (a) between boundary area (12, 51, 52) and ultrasound transducer, wherein the evaluation unit (9) is designed to determine the location of intersection points (S) between the sound pressure distribution curves of the individual elements (E1, E2, E3, E4, E5, E6) or grouped elements and to compare these to the calibration curves in which the intersection points (S) of the curves of the same elements (E1, E2, E3, E4, E5, E6) are located above and below the intersection points (S) in the measured sound pressure distribution curves and to determine the curvature radius (r) by interpolation from the location of intersection points, and the result display unit (10) designed to display the determined curvature radius (r).

2. Assembly according to Claim 1,
wherein the simulation unit (8) is designed to select the sound transducer (3) applicable for the determination with the aid of the simulation unit (8), depending on the test object (4), the materials contained therein, the test setup (5), the distance (a) of the boundary area (12, 51, 52) to be examined and the different curvature radii ($r_i$) that will probably occur.

3. Assembly according to Claim 2,
wherein the individual elements (E1, E2, E3, E4, E5, E6) of the ultrasound transducer (3) have a ring-shaped structure, the form, size and distance of which in relation to each other are defined in such a way that incremental focusing in sufficient focal distances ($f_i$) is possible in front of, on and behind the boundary area (12, 51, 52).

4. Assembly according to Claim 1 to 3,
wherein the ultrasound transducer (3), as a function of the probably occurring distance (a) between the boundary area (12, 51, 52) to be examined and the ultrasound transducer (3) and the resulting focal distances ($f_i$) are either plane or exhibiting elements (E1, E2, E3, E4, E5, E6) which are placed on a calotte.

5. Assembly according to Claim 4,
wherein the ultrasound transducer (3), as transmitter, is equipped with rings of identical surface area for better focusing as individual elements (E1, E2, E3, E4, E5, E6).

6. Assembly according to Claim 4,
wherein in the ultrasound transducer (3) the ring sizes, or the ring sizes created by the summation of the individual overlapping elements (E1, E2, E3, E4, E5, E6), are determined in such a way that the sound pressure differences between the individual rings can be registered.

7. Assembly according to Claim 6,
wherein the ultrasound transducer (3), as transmitter and receiver, is adapted to the measuring conditions with the aid of the simulation unit (8), wherein the parameters of the ultrasound transducer (3) are adapted in relation of the test object (4) with the aid of the results of the simulation unit (8), wherein the ultrasound transducer (3) is selected or designed in such a way, that focusing is possible in the transmission mode and sound pressure distributions can be registered in the receiving mode.

8. Assembly according to Claim 7,
wherein at least one ring-shaped element (E4) in the ultrasound transducer (3) is divided into segments (E41, E42, E43, E44) in order to detect an inclined position of the boundary area (12, 51, 52) for the following adjustment, in order to differentiate between spherical, torical and cylindrical curvatures of the boundary areas (12, 51, 52) as well as, in case of sufficiently strong signals, achieve the determination of two curvature radii (r) of two main axes.

9. Assembly according to one of the above claims,

wherein the ultrasound transducer (3) can be operated with a broadband signal as well as a narrow band signal for the determination of the sound pressure distribution between the individual elements (E1, E2, E3, E4, E5, E6) in form of harmonic wave trains of certain length or in form of short impulses.

10. Assembly according to Claim 1,
wherein the simulation unit (8) is designed to provide calibration curves depending on the test object (4), on the test setup (5), on the ultrasound transducer (3), on different curvature radii ($r_i$) that probably occur, and on various distances ($a_i$) between the ultrasound transducer (3) and the boundary area (12, 51, 52).

11. Assembly according to Claim 1,
wherein the recording unit (7) is designed in such a way that it determines the sound pressure shares on the individual elements (E1, E2, E3, E4, E5, E6) in comparison to the overall sound pressure for the entire ultrasound transducer (3) as a function of the focal distance ($f_i$), wherein a determination of the chronological sequence of the sound pressure for each individual element (E1, E2, E3, E4, E5, E6) in the respective focus ($F_i$), a normalization of the sound pressure of grouped elements of the ultrasound transducer (3) in the respective focal distance ($f_i$) and a recording of the sound pressure distribution curves of the peak sound pressure or the pulse integral of the squared sound pressure as a function of the focal distance ($f_i$) takes place,
whereupon the evaluation unit (9) is designed in such a way that it determines the location of the intersection points (S) in the sound pressure distribution curves of the individual elements (E1, E2, E3, E4, E5, E6) or grouped elements and compares these to the calibration curves and selects the two calibration curves in which the intersection points (S) of the same elements are located above and below the intersection points (S) in the measured sound pressure distribution curves, and determines the curvature radius (r) by interpolation from the location of the intersection points.

12. Assembly according to Claim 11,
wherein the recording unit (7) is designed in such a way that it records the chronological sequence of the signal for each element (E1, E2, E3, E4, E5, E6), so that phase changes of the signal and changes of the signal form can be evaluated.

13. Method for determining the combination of curvature radii (r) and distances (a) to acoustic boundary areas (12, 51, 52) in test objects (4) with ultrasound, by means of an assembly according to Claims 1 to 12, in the following steps:

- Simulation of the reflected sound field on the ultrasound transducer (3) for different curvature radii ($r_i$) on boundary areas (12, 51, 52) to be examined, depending on the specified ultrasound transducer (3), the test object (4), the test setup (5), the impedance differences on the boundary area (12, 51, 52) and the distance ($a_i$) between the ultrasound transducer (3) and the boundary area (12, 51, 52) for the different focal distances ($f_i$) with the aid of a simulation unit (8) and generation of a band of calibration curves for different curvature radii ($r_i$) in form of sound pressure distribution curves and the signal form respectively as a function of focal distances ($f_i$),
- determination of the distances ($a_i$) ($a_l$) between ultrasound transducer (3) and boundary area (12, 51, 52) and the running time of the ultrasound signal by the recording unit (7) and selection of calibration curves,
- incremental shifting of the focus ($F_i$) along the axials (40) of the ultrasound transducer (3) to individual locations in corresponding focal distances ($f_i$) in front of, on and behind the boundary area (12, 51, 52) to be examined, by time-delayed overlapping of wave trains of individual elements (E1, E2, E3, E4, E5, E6) and measurement of the sound wave reflected from the individual elements (E1, E2, E3, E4, E5, E6) for the respective focal distance ($F_i$) by the recording unit (7), as a result of which the sound pressure amplitude for each element (E1, E2, E3, E4, E5, E6) and for the grouped elements, the sound pressure distribution shares to the individual elements (E1, E2, E3, E4, E5, E6) for each focal distance ($f_i$) as well as changes of the signal form are determined, wherein the sound pressure distribution curves are created as a function of the focal distances ($f_i$), and
- comparison of the sound pressure distribution curves determined by measurements to the band of calibration curves for the determined distance (a) and determination of the curvature radius (r) of the boundary area (12, 51, 52), wherein the evaluation unit (9) determines the location of intersection points (S) between the sound pressure distribution curves of the individual elements (E1, E2, E3, E4, E5, E6) or the grouped elements and compares these to the calibration curves and allocates them to two bands of calibration curves in which the intersection points (S) of the same elements are located above and below the intersection points (S) in the measured sound pressure distribution curves, and by determination of the curvature radius (r) by interpolation from the location of intersection points.

14. Method according to Claim 13,

wherein the selection of the ultrasound transducer (3) with the aid of the simulation unit (8) according to the parameters of the test object (4) is performed prior to the start of the method for the determination of the curvature radius.

15. Method according to Claim 13 or 14,
    wherein the simulation unit (8) provides calibration curves depending on the curvature radius (r) of the boundary area (12, 51, 52), the distance (a) between the ultrasound transducer (3) and the boundary area (12, 51, 52) to be examined, the element geometry of the ultrasound transducer (3), the test setup (5) and the test object (4) as a function of the focal distance ($f_i$).

16. Method according to one of the Claims 13 to 15,
    wherein in the recording unit (7) the sound pressure shares on the individual elements (E1, E2, E3, E4, E5, E6) are determined in comparison to the entire ultrasound transducer (3) as a function of the focal distance ($f_i$), wherein a normalization of the sound pressure of the grouped elements of the ultrasound transducer (3) in the respective focal distance ($f_i$) and recording of the sound pressure distribution curves take place, wherein the curvature radius (r) is determined by determining the shifting of the intersection points (S) of the sound pressure distribution curves of the individual elements (E1, E2, E3, E4, E5, E6) or the grouped elements by comparison to the calibration curves.

17. Method according to at least one of the above Claims 13 to 16,
    wherein the effect of the damping effects is minimized by the normalization of the sound pressure of the individual elements (E1, E2, E3, E4, E5, E6) on the sound pressure on the total area (E1+E2+E3+E4+E5+E6) of the ultrasound transducer (3) for he respective focus ($F_i$).

18. Method according to at least one of the above Claims 13 to 17,
    wherein the recording unit (7) also records the time sequence of the signal for each element (E1, E2, E3, E4, E5, E6), so that phase changes of the signal and the signal form can be evaluated.

19. Method according to at least one of the above Claims 13 to 18,
    wherein the determination of the curvature radius (r) and the form of the boundary area (12, 51, 52) and its distance (a) takes place using the ultrasound echo signal at the boundary area (12, 51, 52), wherein the transmitter/receiver of the ultrasound transducer (3) and the boundary area (12, 51, 52) remain adjusted in relation to each other and in the resting position, wherein the reflecting boundary area (12, 51, 52) specified plane as well as curved spherical, torical and/or cylindrical, wherein the curvature radius information is obtained by variation of the transmitted wavefront and by discretization of the sound field at the receiver of the ultrasound transducer (3).

20. Method according to at least one of the above Claims 13 to 18,
    wherein the determination of the curvature radius (r) of the boundary area (12, 51, 52) is performed by simultaneous combined measurement of the distance (a) and the curvature radius (r), including in case of dynamic events such as motion of the boundary area (12, 51, 52), wherein the curvature radius information is obtained by variation of the transmitted wavefront and by discretization of the sound field at the receiver of the ultrasound transducer (3).

21. Method according to at least one of the above Claims 13 to 20,
    wherein the determination of the curvature of a boundary area, in relation to with other boundary areas are located upstream, is performed.

22. Method according to at least one of the above Claims 13 to 21,
    wherein an additional movement of the ultrasound transducer is performed for tracking of bubbles in flow and diffusion processes of liquid media and/or for the expansion of the measuring range, preferably when capturing the curvature path over a range of multiples of the sound beam width.

**Revendications**

1. Agencement (1) et procédé de détermination de la combinaison de rayons d'inclinaison (r) et d'intervalles (a) sur des surfaces limites (12, 51, 52) acoustiques dans des objets de mesure (4) à l'aide d'ultrasons composés de un générateur d'impulsions (2) formé pour générer n>2 signaux d'excitation (13) temporisés l'un par rapport à l'autre

   avec n $\in$ N,
   un aiguillage d'émission et de réception (20),

un transducteur d'ultrasons (3) formé de n>2 avec n $\in$ N éléments (E1, E2, E3, E4, E5, E6) pour émettre et recevoir des ondes acoustiques et pour recevoir les signaux (13) du générateur d'impulsions (2) via l'aiguillage d'émission et de réception (20) et dont les éléments (E1, E2, E3, E4, E5, E6) sont formés pour être excités avec temporisation et qui est également formé pour émettre une onde acoustique (311, 321, 331, 341, 351, 361) temporisée dans l'objet de mesure (4) d'une surface limite (12, 51, 52) d'un objet de mesure (4) à partir de chaque élément (E1, E2, E3, E4, E5, E6) et qui est formé également pour recevoir après commutation sur réception par un interrupteur (21, 22, 23, 24, 25, 26) se trouvant dans l'aiguillage d'émission et de réception (20) les ondes acoustiques réfléchies par la surface limite (12, 51, 52) séparément à chaque élément (E1, E2, E3, E4, E5, E6), un agencement de mesure (5) étant indiqué au moins par le transducteur d'ultrasons (3) et par la direction du balayage acoustique vers l'objets de mesure (4).

n>2, avec n $\in$ N unités d'amplification (6) formés pour recevoir les signaux reçus du transducteur d'ultrasons (3) via l'aiguillage d'émission et de réception (20),

d'une unité d'enregistrement (7) comprenant au moins un convertisseur analogique-numérique pour conversion analogique-numérique des signaux transmis par l'aiguillage d'émission et de réception (20) via les unités d'amplification (6),

une unité d'évaluation (9) placée en aval de l'unité d'enregistrement (7) et

une unité d'affichage de résultats (10) placée en aval de l'unité d'évaluation (9),

le transducteur d'ultrasons (3) étant formé pour déplacer les foyers ($F_i$ avec i=O, 1, 2, ..., m) générés en fonction d'un régime de temporisation sélectionné à partir du chevauchement des séries d'ondes acoustiques (311, 321, 331, 341, 351, 361) des éléments individuels (E1, E2, E3, E4, E5, E6) progressivement le long des axiaux (40) du transducteur d'ultrasons (3) à différents endroits à intervalles de foyer correspondants ($f_i$) et les placer devant, sur et derrière la surface limite (12, 51, 52) à réfléchir,

une unité de simulation (8) formée pour simuler le champ acoustique sur le transducteur d'ultrasons (3) pour différents rayons d'inclinaison ($r_j$) de la surface limite (12, 51, 52) et pour différents intervalles ($a_i$) entre le transducteur d'ultrasons (3) et la surface limite (12, 51, 52) en fonction du transducteur d'ultrasons (3) prévu, de l'objet de mesure (4) et de l'agencement de mesure (5) pour les différents foyers (Fi); pour chaque intervalle ($a_i$) et pour différents rayons d'inclinaison ($r_j$), respectivement un groupe de courbes de calibrage sous la forme de courbes de répartition de pression acoustique étant livré comme fonction des intervalles de foyer ($f_i$) ainsi que la forme de signal, l'unité de simulation (8) étant raccordée à l'unité d'évaluation (9),

l'unité d'enregistrement (7) est formée pour déterminer, outre la intervalles (a) déterminée par la durée entre la surface limite (12, 51, 52) et le transducteur d'ultrasons (3) pour tous les différents foyers ($F_i$), l'onde acoustique réfléchie sur les différents éléments (E1, E2, E3, E4, E5, E6) et d'établir à partir de cela pour chaque élément (E1, E2, E3, E4, E5, E6) et/ou pour des éléments regroupés le déroulement en durée de la pression acoustique et à partir de cela l'amplitude acoustique sur chaque élément ($E_k$) ainsi que les parts de répartition de pression acoustique sur les différents éléments (E1, E2, E3, E4, E5, E6) pour chaque foyer ($F_i$) ainsi les courbes de répartition de pression acoustique comme fonction des intervalles focaux ($f_i$), et

l'unité d'évaluation (9) est formée pour comparer les courbes de répartition de pression acoustique obtenues par mesure avec la succession des courbes de calibrage pour la intervalles (a) calculée entre la surface limite (12, 51, 52) et le transducteur d'ultrasons (3), l'unité d'évaluation (9) étant formée pour déterminer la position des points d'intersection (S) entre les courbes de répartition de pression acoustique des éléments (E1, E2, E3, E4, E5, E6) ou des éléments regroupés et pour sélectionner les deux successions de courbes de calibrage pour lesquelles les points d'intersection (S) des courbes des mêmes éléments (E1, E2, E3, E4, E5, E6) se trouvent au-dessus et en dessous des points d'intersection (S) dans les courbes de répartition de pression acoustique et pour déterminer le rayon d'inclinaison (r) par interpolation à partir de la position des points d'intersection et l'unité d'affichage des résultats (10) est formée pour indiquer le rayon d'inclinaison (r) déterminé.

2. Agencement selon la revendication 1,
l'unité de simulation (8) étant formée pour sélectionner le transducteur d'ultrasons (3) concerné pour la détermination à l'aide de l'unité de simulation (8) en fonction de l'objets de mesure (4), des matériaux y étant contenus, de l'agencement de mesure (5), de l'intervalle (a) de la surface limite (12, 51, 52) à examiner et des différents rayons d'inclinaison ($r_j$) qui apparaîtront probablement.

3. Agencement selon la revendication 2,
les différents éléments (E1, E2, E3, E4, E5, E6) du transducteur d'ultrasons (3) possédant une structure annulaire

EP 2 090 229 B1

dont la forme, la taille et les intervalles entre eux sont fixées de façon qu'une concentration progressive soit possible à intervalle focal ($f_i$) suffisante devant, sur et derrière la surface limite (12, 51, 52).

4. Agencement selon la revendication 1 à 3, le transducteur d'ultrasons (3) étant soit plat soit présentant des éléments (E1, E2, E3, E4, E5, E6) placés sur une calotte en fonction de l'intervalle (a) apparaissant probablement entre la surface limite (12, 51, 52) à examiner et le transducteur d'ultrasons (3) et les intervalles focaux ($f_i$) en résultant.

5. Agencement selon la revendication 4, le transducteur d'ultrasons (3) en tant qu'émetteur possédant des anneaux de même surface pour une meilleure concentration comme éléments (E1, E2, E3, E4, E5, E6).

6. Agencement selon la revendication 4, pour le transducteur d'ultrasons (3), les tailles d'anneau ou les tailles d'anneau générées par l'addition via les différents éléments (E1, E2, E3, E4, E5, E6) juxtaposés étant fixées de façon qu'il soit possible d'enregistrer les différences de pression acoustique entre les différents anneaux.

7. Agencement selon la revendication 6, le transducteur d'ultrasons (3) en tant qu'émetteur et récepteur, connaissant une adaptation des conditions de mesure à l'aide de l'unité de simulation (8), les paramètres du transducteur d'ultrasons (3) étant adaptés en référence de l'objets de mesure (4) à l'aide des résultats de l'unité de simulation (8), le transducteur d'ultrasons (3) étant sélectionné ou fabriqué de façon qu'en mode émission, une concentration soit possible et en mode réception, les répartitions de pression acoustique puissent être enregistrées.

8. Agencement selon la revendication 7, au moins un élément annulaire (E4) étant réparti en segments (E41, E42, E43, E44) dans le transducteur d'ultrasons (3) pour détecter une position oblique de la surface pour un ajustage suivant pour différencier des inclinaisons sphériques, toriques et cylindriques des surfaces limites (12, 51, 52) ainsi qu'en cas de signaux suffisamment important pour atteindre une détermination de deux rayons d'inclinaison (r) des axes principaux.

9. Aménagement selon l'une des revendications précédentes, le transducteur d'ultrasons (3) étant exploitable tant avec un signal à large bande qu'avec un signal à bande étroite pour déterminer la répartition de pression acoustique entre les différents éléments (E1, E2, E3, E4, E5, E6) sous la forme de séries d'ondes harmoniques d'une certaine longueur ou sous la forme d'impulsions courtes.

10. Aménagement selon la revendication 1, l'unité de simulation (8) étant formée pour mettre à disposition des courbes de calibrage en fonction de l'objets de mesure (4), de l'agencement de mesure (5), du transducteur d'ultrasons (3), des rayons d'inclinaison ($r_j$) apparaissant probablement, des différents intervalles ($a_i$) entre le transducteur d'ultrasons (3) et la surface limite (12, 51, 52).

11. Aménagement selon la revendication 1, l'unité d'enregistrement (7) étant formée de sorte qu'elle détermine les parts de pression acoustique sur les éléments (E1, E2, E3, E4, E5, E6) en comparaison a la pression acoustique globale pour le transducteur d'ultrasons (3) comme fonction de l'intervalle focal ($f_i$), et parallèlement une détermination du déroulement en durée de la pression acoustique ayant lieu pour chaque éléments (E1, E2, E3, E4, E5, E6) dans le foyer respectif ($F_i$), un cadrage sur la pression acoustique des éléments regroupés du transducteur d'ultrasons (3) ayant lieu dans l'intervalle focal ($f_i$) et un enregistrement des courbes de répartition de pression acoustique de la pression acoustique de pointe ou de l'intégrale d'impulsion de la pression acoustique élevée au carrée ayant lieu comme fonction de l'intervalle focal ($f_i$) et l'unité d'évaluation (9) étant formée de façon qu'elle détermine la position des intersections (S) dans les courbes de répartition de pression acoustique des éléments (E1, E2, E3, E4, E5, E6) ou d'éléments regroupés et compare avec les courbes de calibrage et choisit les deux courbes de calibrage où les points d'intersection (S) des mêmes éléments se trouvent au-dessus et en dessous des points d'intersections (S) dans les courbes de répartition de pression acoustique mesurées et détermine le rayon d'inclinaison (r) par interpolation à partir de la position des points d'intersection.

12. Aménagement selon la revendication 11, L'unité d'enregistrement (7) étant formée de façon à enregistrer le déroulement dans la durée du signal pour chaque élément (E1, E2, E3, E4, E5, E6) de façon que les modifications de phase du signal et la modification de forme du signal puissent être évaluées.

**13.** Procédé de détermination de la combinaison des rayons d'inclinaison (r) et des intervalles (a) aux surfaces limites (12, 51, 52) dans des objets de mesure (4) par ultrasons avec l'aménagement selon les revendications 1 à 12 avec les opérations suivantes:

- simulation du champ acoustique réfléchi sur le transducteur d'ultrasons (3) pour différentes rayons d'inclinaison ($r_j$) de la surface limite (12, 51, 52) à examiner en fonction du transducteur d'ultrasons (3) prévu, de l'objet de mesure (4), de l'agencement de mesure (5), des différences d'impédance à la surface limite (12, 51, 52) et de l'intervalle ($a_i$) entre le transducteur d'ultrasons (3) et la surface limite (12, 51, 52) pour les différents intervalles de foyer ($f_i$) à l'aide d'une unité de simulation (8) et génération d'une succession de courbes de calibrage pour différents rayons d'inclinaison (r) sous la forme de courbes de répartition de pression acoustique et des formes de signaux respectivement comme fonction des intervalles de foyer ($f_i$),
- détermination des intervalles ($a_i$) entre le transducteur d'ultrasons (3) et la surface limite (12, 51, 52) à partir de la durée du signal à ultrasons par l'unité d'enregistrement (7) et choix des courbes de calibrage,
- déplacement progressif des foyers ($F_i$) le long des axiaux (40) du transducteur d'ultrasons (3) à différents endroits à intervalles de foyer correspondants ($f_i$) devant, sur et derrière la surface limite (12, 51, 52) à réfléchir par superposition temporisée des séries d'ondes des éléments (E1, E2, E3, E4, E5, E6) et mesure de l'onde acoustique réfléchie pour l'intervalle de foyer ($F_i$) respectif sur les différents éléments (E1, E2, E3, E4, E5, E6) par l'unité d'enregistrement (7) à partir de quoi les amplitudes de pression acoustique pour chaque élément (E1, E2, E3, E4, E5, E6) et pour les éléments regroupés détermine les parts de répartition de pression acoustique sur les différents éléments (E1, E2, E3, E4, E5, E6) pour chaque intervalle de foyer ($f_i$) ainsi que les modifications de forme de signal, les courbes de répartition de pression acoustique étant réalisées comme fonction des intervalles de foyer ($f_i$), et
- comparaison des courbes de répartition de pression acoustique déterminées par mesure avec la succession des courbes de calibrage pour l'intervalle (a) obtenu et détermination du rayon d'inclinaison (r) de la surface limite (12, 51, 52), l'unité d'évaluation (9) déterminant la position des intersections (S) entre les courbes de répartition de pression acoustique des éléments (E1, E2, E3, E4, E5, E6) ou d'éléments regroupés et compare avec les courbes de calibrage et affecte aux deux successions de courbes de calibrage où les points d'intersection (S) des courbes des mêmes éléments se trouvent au-dessus et en dessous des points d'intersections (S) dans les courbes de répartition de pression acoustique mesurées et détermine le rayon d'inclinaison (r) par interpolation à partir de la position des points d'intersection, et
- édition du rayon d'inclinaison (r) déterminé à l'unité d'affichage des résultats (10).

**14.** Procédé selon la revendication 13,
Le choix du transducteur d'ultrasons (3) s'effectuant à l'aide de l'unité de simulation (8) conformément aux paramètres de l'objet de mesure (4) avant le début du procédé de détermination du rayon d'inclinaison.

**15.** Procédé selon la revendication 13 ou 14,
L'unité de simulation (8) fournissant des courbes de calibrage en fonction du rayon d'inclinaison (r) de la surface limite (12, 51, 52), de l'intervalle (a) entre le transducteur d'ultrasons (3) et la surface limite (12, 51, 52) à examiner, de la, géométrie d'élément du transducteur d'ultrasons (3), de l'agencement de mesure (5) et de l'objet de mesure (4) comme fonction de l'intervalle de foyer ($f_i$).

**16.** Procédé selon l'une des revendications 13 à 15,
dans l'unité d'enregistrement (7), les parts de pression acoustique sur les éléments (E1, E2, E3, E4, E5, E6) étant déterminées en comparaison à la pression acoustique globale pour l'ensemble du transducteur d'ultrasons (3) comme fonction de l'intervalle de foyer ($f_i$), un cadrage sur la pression acoustique des éléments regroupés du transducteur d'ultrasons (3) s'effectuant dans l'intervalle de foyer ($f_i$) respectif et un enregistrement des courbes de répartition de pression acoustique s'effectuant, la détermination du déplacement des points d'intersections (S) des courbes de répartition de pression acoustique des éléments (E1, E2, E3, E4, E5, E6) ou des éléments regroupés en comparaison aux courbes de calibrage permettant de déterminer le rayon d'inclinaison (r).

**17.** Procédé selon au moins l'une des revendications 13 à 16,
le cadrage de la pression acoustique des éléments (E1, E2, E3, E4, E5, E6) sur la pression acoustique de la surface totale (E1 + E2 + E3 + E4 + E5 + E6) du transducteur d'ultrasons (3) pour le foyer ($F_i$) permettant de diminuer l'impact des effets d'amortissement.

18. Procédé selon au moins l'une des revendications 13 à 17,
l'unité d'enregistrement (7) enregistrant de plus le déroulement de durée du signal pour chaque élément (E1, E2, E3, E4, E5, E6) de façon que les modifications de phase du signal et la modification de forme de signal puissent être évaluées.

19. Procédé selon au moins l'une des revendications 13 à 18,
la détermination du rayons d'inclinaison (r) et la forme de la surface limite (12, 51, 52) et leur intervalle (a) étant effectuée à partir du signal d'écho à ultrasons pour les surfaces limites (12, 51, 52), l'émetteur/récepteur du transducteur d'ultrasons (3) et la surface limite (12, 51, 52) étant constamment ajustés l'un par rapport à l'autre et restant en position de repos, la surface limite (12, 51, 52) réfléchissante étant donnée plane ainsi que sphérique, torique et/ou inclinée de façon cylindrique, l'information de rayon d'inclinaison étant obtenue par la variation du front d'ondes envoyé et par discrétisation du champ acoustique au récepteur du transducteur d'ultrasons (3).

20. Procédé selon au moins l'une des revendications 13 à 18,
une mesure combinée parallèle de l'intervalle (a) et du rayon d'inclinaison (r) permettant de déterminer le rayons d'inclinaison (r) de la surface limite (12, 51, 52) même en cas d'opérations dynamiques - lors du mouvement de la surface limite (12, 51, 52) -, l'information de rayon d'inclinaison étant obtenue par la variation du front d'ondes envoyé et par discrétisation du champ acoustique au récepteur du transducteur d'ultrasons (3).

21. Procédé selon au moins l'une des revendications 13 à 20,
l'inclinaison d'une surface limite en amont de laquelle sont placées d'autres surfaces limites étant déterminée.

22. Procédé selon au moins l'une des revendications 13 à 21,
un mouvement supplémentaire de transducteur d'ultrasons étant effectué pour suivre les bulles dans les processus de flux et de diffusion des agents liquides et/ou pour agrandir la zone de mesure, de préférence lors de la saisie d'un développement d'inclinaison sur une zone représentant un multiple de la largeur du faisceau acoustique.

# Fig. 1

Fig. 1a

Fig. 1b

Fig. 2

# Fig. 3

Fig. 3a

Fig. 3b

Fig. 3c

Fig. 4a

Fig. 4b

Fig. 4c

Fig. 4

EP 2 090 229 B1

## Fig. 5

Fig. 5a

Fig. 5b

Fig. 5c

Fig. 6

EP 2 090 229 B1

Fig. 7

a=10 mm, $r_2$=10 mm

Druckverteilung V

Fokusabstand f [mm]

E1
E2
E3
E4
E5
E6

**a=10 mm, r =9 mm**

Fig. 8

EP 2 090 229 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5345939 A


**In der Beschreibung aufgeführte Nicht-Patentliteratur**

- **Druckschrift Volker Deutsch ; Michael Platte.** Manfred Vogt: Ultraschallprüfung. Springer Verlag, 1997, 28, 29
- **Akhnak M. et al.** Development of a segmented annular array transducer for acoustic imaging. *NDT&E International 35,* 2002, vol. 35 (7), ISSN 0963-8695, 427-431
- **Barshan Billur et al.** Radius of curvature estimation and localization of targets using multiple sonar sensors. *Journal of the Acoustical Society of America,* April 1999, 2318-2331
- **Druckschrift: Volker Deutsch ; Michael Platte ; Manfred Vogt.** Ultraschallprüfung. Springer Verlag, 1997, 133ff
- Pulse-echo techniques, Quality of linear and sector scan images and the compound scan. **Druckschrift Hertz, C.H. et al.** Handbook of clinical ultrasound. John Wiley & Sons, 1978, 35-36
- **Druckschrift Thijssen, J.M.** A new B-scan system for ophthalmic diagnosis. *Ophthalmic Research,* 1971, vol. 2, 285-294
- Physik und Technik der Ultraschalldiagnoseverfahren. **Druckschrift Klews, P.-M.** Moderne Bildgebung. Thieme, 1998, 197-221
- **B. Barshan et al.** Radius of curvature estimation and localization of targets using multiple sonar sensors. *J. Acoust. Soc. Am.,* April 1999, vol. 105 (4), 2318-2331
- Druckschrift: Plane arrays - Fundamental investigations for correct steering by means of sound field calculations. *Wave Motion,* 2007, vol. 44, 248-261
- **Druckschrift Kühnicke, E.** Optimization of ultrasound broadband transducers for complex testing problems by means of transient and time harmonic sound fields. *9th European Conference on NDT,* September 2006